# EUROPÄISCHE PATENTSCHRIFT

(11) **EP 1 866 272 B1**
(45) Veröffentlichungstag und Bekanntmachung des Hinweises auf die Patenterteilung: **18.10.2017**
(21) Anmeldenummer: 06707335.3
(22) Anmeldetag: 28.02.2006
(51) Int. Cl.: C07C 51/25, C07C 45/52, C08F 220/06, C08L 33/02, B01J 8/02

(54) **AUF NACHWACHSENDEN ROHSTOFFEN BASIERENDE ACRYLSÄURE UND WASSERABSORBIERENDE POLYMERGEBILDE SOWIE VERFAHREN ZU DEREN HERSTELLUNG**
ACRYLIC ACID, WATER-ABSORBENT POLYMER STRUCTURES BASED ON RENEWABLE RESOURCES AND METHOD FOR PRODUCING SAID STRUCTURES
ACIDE ACRYLIQUE ET STRUCTURES POLYMERES ABSORBANT L'EAU A BASE DE MATIERES PREMIERES RENOUVELABLES ET PROCEDE DE FABRICATION

(30) Priorität: 28.02.2005 DE 102005009586
(43) Veröffentlichungstag der Anmeldung: 19.12.2007
(73) Patentinhaber: Evonik Degussa GmbH, 45128 Essen (DE)
(72) Erfinder: BUB, Günther, 45772 Marl (DE); MOSLER, Jürgen, 44577 Castrop-Rauxel (DE); SABBAGH, Andreas, 48249 Dülmen (DE); KUPPINGER, Franz-Felix, 45768 Marl (DE); NORDHOFF, Stefan, 45657 Recklinghausen (DE); STOCHNIOL, Guido, 45721 Haltern (DE); SAUER, Jörg, 48249 Dülmen (DE); KNIPPENBERG, Udo, 45772 Marl (DE); LATOSCHINSKI, Günter, 45770 Marl (DE); FURNO, Franck, 40545 Düsseldorf (DE); FRICKE, Michael Bernd, 48712 Hockmoor (DE); ZANTHOFF, Horst-Werner, 45481 Mülhiem an der Ruhr (DE)
(74) Vertreter: Lang, Arne
(86) Internationale Anmeldenummer: PCT/EP2006/001831
(87) Internationale Veröffentlichungsnummer: WO 2006/092272

(56) Entgegenhaltungen:
- EP-A1- 1 319 648
- EP-A2- 0 890 591
- WO-A2-02/40039
- DE-A1- 10 138 150
- DE-C1- 4 238 493
- US-A- 1 916 743
- US-A1- 2002 120 085
- US-B1- 6 727 383
- DATABASE WPI Section Ch, Week 200560 Derwent Publications Ltd., London, GB; Class A41, AN 2005-585659 XP002384230 & JP 2005 213225 A (NIPPON SHOKUBAI CO LTD) 11. August 2005 (2005-08-11)

## Beschreibung

Die vorliegende Erfindung betrifft ein Verfahren zur Herstellung von Acrylsäure, ein Verfahren zur Herstellung von Polymeren durch radikalische Polymerisation von Acrylsäure, vorzugsweise zur Herstellung wasserabsorbierender Polymere, und eine Vorrichtung zur Herstellung von Acrylsäure, und offenbart die durch dieses Verfahren erhältliche Acrylsäure sowie die durch dieses Verfahren erhältlichen wasserabsorbierenden Polymere, wasserabsorbierende Polymere, welche zu mindestens 25 Gew.-% auf teilneutralisierter Acrylsäure basieren, einen Verbund, ein Verfahren zur Herstellung eines Verbundes, den durch dieses Verfahren erhältlichen Verbund und die Verwendung von Acrylsäure zur Herstellung wasserabsorbierender Polymergebilde, sowie.

An die Reinheit von Acrylsäure, welche zur Herstellung polymerer Verbindungen eingesetzt werden, werden hohe Anforderungen gestellt. Dieses gilt insbesondere dann, wenn es sich bei den Polymeren um so genannte Superabsorber handelt, die in Wundauflagen oder Hygieneartikel eingearbeitet werden. Diese Polymere sind in der Lage, wässrige Flüssigkeiten unter Bildung eines Hydrogels zu absorbieren und damit zu binden. Superabsorber finden daher insbesondere in Hygieneartikeln wie Windeln, Inkontinenzeinlagen, Damenbinden und dergleichen zur Absorption von Körperflüssigkeiten Verwendung. Einen umfassenden Überblick über Superabsorber, ihre Anwendung und ihre Herstellung geben F. L. Buchholz und A. T. Graham (Herausgeber) in "Modern Superabsorbent Polymer Technology", Wiley-VCH, New York, 1998.

Zur Herstellung der superabsorbierenden Polymere wird üblicherweise eine Acrylsäure eingesetzt, welche durch katalytische Gasphasenoxidation von Propylen zu Acrolein, das dann in einer weiteren katalytischen Gasphasenoxidation zu Acrylsäure, anschließende Absorption des gasförmigen Reaktionsgemisches in Wasser, Destillation der so erhaltenen wässrigen Acrylsäurelösung unter Erhalt einer Roh-Acrylsäure und weitere Aufreinigung der Roh-Acrylsäure mittels Destillation oder Kristallisation als Rein-Acrylsäure erhalten wird.

Nachteilig bei diesem Verfahren zur Herstellung von Acrylsäure ist. dass die in beiden Stufen angewandten Temperaturen zwischen 300 und 450°C zur Bildung von Oligomeren und weiteren unerwünschten Crackprodukten führen. Dieses hat zur Folge, dass eine unerwünscht hohe Menge an höher als die Acrylsäure siedenden Verbindungen oder von Acrylsäure nur schwer trennbaren Verbindungen wie Essigsäure anfallen, die von der Acrylsäure abgetrennt werden müssen. Diese in der Regel destillativ erfolgende Abtrennung führt zu einer weiteren thermischen Belastung der Acrylsäure, welche die nachteilige Bildung von dimerer oder oligomerer Acrylsäure begünstigt. Ein hoher Gehalt an Acrylsäuredimeren oder Acrylsäureoligomeren ist jedoch nachteilig, da diese Dimeren oder Oligomeren bei der Herstellung von Superabsorbern durch radikalische Polymerisation von Acrylsäure in Gegenwart von Vernetzern in das Polymerrückgrad eingebaut werden. Bei der im Anschluss an die Polymerisation erfolgenden Nachbehandlung der Oberfläche der Polymerpartikel, beispielsweise im Rahmen einer Oberflächennachvemetzung, werden die einpolymerisierten Dimere jedoch unter Bildung von β-Hydroxypropionsäure gespalten, die unter den Nachvernetzungsbedingungen unter Bildung von Acrylsäure dehydratisiert wird. Ein hoher Gehalt an dimerer Acrylsäure in der zur Herstellung der Superabsorber eingesetzten Acrylsäure führt daher dazu, dass der Gehalt an Acrylsäuremonomeren bei einer thermischen Behandlung der Polymere, wie sie bei der Nachvernetzung erfolgt, ansteigt.

Da die löslichen Anteile, insbesondere die Acrylsäuremonomere in den superabsorbierenden Polymeren Hautreizungen hervorrufen können, setzt ein Einsatz dieser Polymere in Hygieneartikeln einen besonders geringen Gehalt an extrahierbaren Bestandteilen voraus.

Auch andere, oftmals toxische Verbindungen sind in der durch die katalytische Gasphasenoxidation erhältlichen Acrylsäure noch enthalten. Zu diesen Verunreinigungen gehören insbesondere Aldehyde, die sich störend auf den Polymerisationsverlauf auswirken, mit der Folge, dass die Polymere noch beachtliche Mengen an löslichen Bestandteilen beinhalten.

Auf herkömmliche Weise aus Propylen hergestellte Acrylsäure enthält nicht unerhebliche Mengen an Doppelbindungen aufweisenden Ketonen, insbesondere an Protoanemonin (PTA). Diese Verbindung kann bei einem Kontakt mit der Haut Vergiftungserscheinungen, wie etwa Rötung, Juckreiz oder Blasenbildung, hervorrufen. Auf Acrylsäure basierende Superabsorber, welche hohe Mengen an PTA als lösliche Bestandteile beinhalten, sind daher aus dermatologischer Sicht bedenklich. Außerdem stört PTA die Polymerisation, wie dies etwa in der US-A-2002/0120085 beschrieben ist. Dieses führt zum Erhalt von superabsorbierenden Polymeren mit weniger guten Saug-, Transport- und Rückhalteeigenschaften von Körperflüssigkeiten, so dass bei Verwendung von derartigen superabsorbierenden Polymeren in Hygieneartikeln wie Windeln oder Damenbinden ein schlechterer Tragekomfort beispielsweise durch "Leakage" auftritt.

Im Stand der Technik sind bereits einige Verfahren beschrieben worden, mit denen der Gehalt an den vorstehend genannten Verbindungen, insbesondere von Aldehyden oder PTA, in der durch Gasphasenoxidation von Propylen erhaltenen Acrylsäure reduziert werden kann.

US 2002/120085 A1 und EP 0 890 591 A2 offenbaren wasserabsorbierende Polymergebilde von Acrylsäure und deren Verwendung.

JP 2005-213225 A beschreibt eine Methode zur Herstellung von Acrylsäure unter Einsatz von Rohstoffen, die nicht von Erdöl abhängen.

US 6 727 383 B1 und EP 1 319 648 A1 beschreiben die Gasphasenoxidation von Acrolein unter Erhalt eines Acrylsäure aufweisenden Monomergas, das Quenchen des Monomergases und die Aufarbeitung der Quenchphase.

DE 42 38 493 C1 und US 1 916 743 beschreiben die Dehydratisierung von Glycerin zu Acrolein.

WO 02/40039 A2 beschreibt die Verwendung von superabsorbierenden Acrylsäure Polymeren zur Entfernung von Flüssigkeit aus dem Darmtrakt eines Tieres oder Menschen.

DE-A-101 38 150 A1 schlägt zur Verminderung des Aldehyd-Anteils in der Acrylsäure vor, diese mit einem Aldehydfänger in Kontakt zu bringen, um die Aldehyde in Hochsiedende Verbindungen zu überführen, die dann mittels Destillation abgetrennt werden können.

Zur Entfernung des PTAs werden im Stand der Technik verschiedene Verfahren vorgeschlagen, wie etwa die Zugabe eines Salzes der salpetrigen Säure, von Stickstoffoxid oder von Nitrobenzol (JP 81-41614) oder die Zugabe eines oder mehrerer para-Phenylendiamine (EP-A-567 207) zur Acrylsäure.

Der Nachteil der vorstehend beschriebenen Verfahren zur Verminderung des Anteils an Aldehyden und Ketonen in Acrylsäure besteht jedoch unter anderem darin, dass, sofern der Gehalt an Verunreinigungen in der Acrylsäure nicht genau bekannt ist, diese Reagenzien zum Zwecke einer möglichst vollständigen Entfernung der Verunreinigungen aus der Acrylsäure im Überschuss eingesetzt werden müssen. Zum einen der Acrylsäure reaktive Reagenzien zugesetzt werden müssen. Der nicht umgesetzte Teil dieser Reagenzien muss anschließend wieder entfernt werden. Nicht entfernte Reagenzien sind in den aus einer solchen Acrylsäure erhaltenen Superabsorbent als lösliche Bestandteile enthalten, die bei einem Einsatz der Superabsorber in Hygieneartikeln in Kontakt mit der Haut des Hygieneartikelträgers kommen können. Weiterhin lassen sich mit den aus dem Stand der Technik bekannten Verfahren zur Entfernung von Aldehyden und Ketonen aus Acrylsäure diese Verunreinigungen nur selten vollständig entfernen. Außerdem können sich derartige Zusätze nachteilig auf die Polymerisation und damit auf die Eigenschaften des daraus erhaltenen Polymers auswirken.

Neben den Nachteilen, die auf Verunreinigungen in der zur Herstellung von Superabsorbern eingesetzten Acrylsäure zurückzuführen sind, weisen herkömmliche Superabsorber auch den Nachteil auf, dass sie, sofern sie nicht zumindest anteilig natürliche Polymere, wie etwa Zellulose, beinhalten, kaum auf nachwachsenden Rohstoffen basieren. Zwar ist es gelungen, viele der in Hygieneartikeln, insbesondere in Wegwerfwindeln, eingesetzten Komponenten aus biologischen Ausgangsstoffen herzustellen, doch ist ein Ersatz der auf vernetzten Polyacrylaten basierenden Superabsorber durch natürliche, superabsorbierende Polymere, wie etwa durch vernetzte, derivatisierte Stärke oder Zellulose, in der Regel mit signifikanten Einbußen hinsichtlich der Absorbereigenschaften verbunden. Dieses führt meist dazu, dass, um auch nur annährend die gleichen Absorbereigenschaften in einem Hygieneartikel zu erreichen, wesentlich mehr dieser auf natürlichen Polymeren basierenden Absorber eingesetzt werden müssen. Dieses ist nachteilig, weil die Hygieneartikel dadurch voluminöser und schwerer werden, was den Tragekomfort deutlich einschränkt und zu einem größeren Abfallvolumen führt, das neben mehr Deponieraum bzw. Verbrennungsaufwand mehr Transportkapazitäten für die Abfallentsorgung notwendig macht. All dieses wirkt sich nachteilig auf die Umweltverträglichkeit der auf natürlichen Polymeren basierenden Absorber aus.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, Polymere, insbesondere Superabsorber, bereitzustellen, welche einen besonders geringen Gehalt an extrahierbaren, gegebenenfalls toxischen Bestandteilen aufweisen.

Weiterhin lag der vorliegenden Erfindung die Aufgabe zugrunde, Polymere, insbesondere Superabsorber, bereitzustellen, die umweltverträglich sind und dennoch über hervorragende Anwendungseigenschaften verfügen. So galt es insbesondere, Superabsorber mit einer verbesserten Umweltverträglichkeit bei gleich bleibend guten Absorbereigenschaften zu schaffen.

Zudem war es eine Aufgabe der vorliegenden Erfindung, die Umweltverträglichkeit der die erfindungsgemäßen Polymere beinhaltenden Weiterverarbeitungsprodukte, wie Verbunde allgemein und Hygieneartikel im Besonderen, zu verbessern, ohne dass die gewünschten Funktionen, wie Saugfähigkeit, Tragekomfort und einfache Herstellbarkeit dieser Weiterverarbeitungsprodukte darunter leiden.

Auch lag der vorliegenden Erfindung die Aufgabe zugrunde, ein Verfahren zur Herstellung derartiger Polymere und der zur deren Herstellung geeigneten Monomere anzugeben, wobei dieses Verfahren möglichst ohne den Einsatz reaktiver Verbindungen zur Entfernung von Verunreinigungen aus den zur Herstellung der Polymere eingesetzten Monomere auskommen soll.

Zudem lag eine Aufgabe der vorliegenden Erfindung darin, ein Verfahren und eine Vorrichtung zur Herstellung der Monomere bzw. der Polymere vorzuschlagen, die mit einem möglichst geringen Umrüstaufwand in bestehende großtechnische Fertigungsverfahren und -vorrichtungen integriert werden kann.

Einen Beitrag zur Lösung der vorstehend genannten Aufgaben leistet ein Verfahren zur Herstellung von Acrylsäure, mindestens die folgenden Schritte aufweisend:
a. Dehydratisieren von Glycerin zu einem Acrolein aufweisenden Dehydratisierungsprodukt;
b. Gasphasenoxidation des Dehydratisierungsprodukts unter Erhalt eines Acrylsäure aufweisenden Monomergases;
c. in Kontakt bringen des Monomergases mit einem Quenchmittel unter Erhalt einer Acrylsäure aufweisenden Quenchphase;
d. Aufarbeiten der Quenchphase unter Erhalt einer Acrylsäure beinhaltenden Monomerphase.

Einen weiteren Beitrag zur Lösung der vorstehenden Aufgaben leistet ein Verfahren zur Herstellung eines Polymers durch radikalische Polymerisation von Acrylsäure, mindestens die folgenden Schritte aufweisend:
A. Dehydratisieren von Glycerin zu einem Acrolein aufweisenden Dehydratisierungsprodukt;
B. Gasphasenoxidation des Dehydratisierungsprodukts unter Erhalt eines Acrylsäure aufweisenden Monomergas;
C. in Kontakt bringen des Monomergas mit einem Quenchmittel unter Erhalt einer Acrylsäure aufweisenden Quenchphase;
D. Aufarbeiten der Quenchphase unter Erhalt einer Acrylsäure beinhaltenden Monomerphase;
E. Polymerisation der Monomerphase.

In einer Ausgestaltung des erfindungsgemäßen Verfahrens ist bevorzugt, dass das Glycerin aus der Verseifung von Fetten gewonnen wird. Bei diesen Fetten kann es sich sowohl um tierische als auch um pflanzliche Fette handeln. Tierische Fette fallen insbesondere bei der Tierkörperverwertung an. Pflanzliche Fette fallen in großen Mangen bei der Ölgewinnung aus Ölfrüchten wie Raps, Soja, Sesam, Oliven und Sonnenblumenkernen an. Große Mengen von Glycerin fallen insbesondere bei der Herstellung von sogenanntem "Biodiesel" aus Rapsöl an, wie WO-A-2004/029016 unter anderem zu entnehmen ist. Folglich ist es in dem erfindungsgemäßen Verfahren bevorzugt, dass das Glycerin bei der Erzeugung von flüssigen Brennstoffen aus natürlichen Rohstoffen anfällt. Dieses ist insbesondere bei Ölmühlen nachgeschalteten Verseifungseinrichtungen gegeben.

In einer Ausgestaltung des erfindungsgemäßen Verfahrens ist es bevorzugt, dass die Dehydratisierung mindestens teilweise in flüssiger Phase erfolgt. Als flüssige Phasen sind insbesondere wässrige Systeme bevorzugt. Wenn die Dehydratisierung wenigstens teilweise oder gar vollständig in einer flüssigen Phase durchgeführt wird, hat dieses, insbesondere wenn dies eine wässrige Phase ist, den Vorteil, dass bei hohen Glycerinkonzentrationen hohe Acroleinkonzentrationen in der wässrigen Phase erreicht werden können. Diese wässrigen Phasen mit hohen Acroleinkonzentrationen können direkt in dem nächsten Schritt der Gasphasenoxidation eingesetzt werden. Um den Oxidationskatalysator vor Verkohlung zu schützne, ist es bevorzugt, eine Trenneinheit zwischen Dehydratisierung und Oxidation vorzusehen. In dieser Trenneinheit werden die von Acrolein verschiednen Begleitstoffe abgetrennt und das Acrolein der Gasphasenoxidation zugeführt. So könne wesentlich länger Katalysatorstandzeiten erreicht werden. Ein weiterer Vorteil der Flüssigphasendehydratisierung besteht darin, dass durch die flüssige Phase eine Spülwirkung erzielt werden kann, mit der eine Belagbildung auf einem Feststoffkatalysator deutlich reduziert werden kann, was zu höheren Katalysatorlaufzeiten und damit zu einem geringeren Regenerierungsbedarf des Katalysators führt. Ein weiterer Vorteil der Flüssigkeitsphasendehydratisierung besteht darin, dass diese bei verhältnismäßig moderaten Temperaturen, in einem Bereich von 160 bis 270°C, bevorzugt in einem Bereich von 180 bis 260°C und darüber hinaus bevorzugt in einem Bereich von 215 bis 245°C durchgeführt werden kann. Diese Temperaturbereiche liegen deutlich unter der Zersetzungs- und Siedetemperatur des Glycerins von etwa 290°C, was zur Verringerung von Sumpf- und Crackprodukten sowie anderen Verunreinigungen führt, die sich nachteilig auf die Betriebsdauer der Gasphasenoxidation auswirken. Es ist jedoch in einer weiteren Ausgestaltung des erfindungsgemäßen Verfahrens vorgesehen, die Dehydratisierung in der Nähe des Zersetzungspunkte des Glycerins durchzuführen, um die Ausbeute zu steigern. In dieser Ausgestaltung liegen die Temperaturen in einem Bereich von 170 bis 290°C, bevorzugt in einem Bereich von 190 bis 289°C und darüber hinaus bevorzugt in einem Bereich von 225 bis 288°C. Bei der Flüssigphasendehydratisierung ist es bevorzugt, dass diese in einer Kreisfahrweise erfolgt, bei der im Fall eines Feststoffkatalysators die glycerinhaltige flüssige Phase durch eine Pumpe über den Katalysator in einem Drucksystem geführt wird. Bei der Flüssigkatalyse bzw. homogenen Katalyse wird mindestens ein Teilstrom aus dem Reaktor im Kreis gefahren. Umgesetztes Glycerin, verbrauchter Katalysator und ggf. entzogenes Wasser werden dem in den Reaktor bei der Kreisfahrweise wieder eingespeisten Teilstrom vorzugsweise am Reaktoreingang zugesetzt. Sollte der Teilstrom bei einer sauer homogen katalysierten Dehydratisierung, anfallen, ist es vorteilhaft, dass der Teilstrom zumindest teilweise neutralisiert wird. Durch diese Maßnahme kann die Bildung von Nebenprodukten durch Reaktionen in dem Teilstrom gehemmt oder gar verhindert werden. Ferner ist es vorteilhaft, aus dem Teilstrom höher als Acrolein siedende, als Hochsieder bezeichnete Stoffe abzureichern. Dieses kann beispielsweise durch Membranen, die vorzugsweise semipermeabel sind, aufweisende Trenneinheiten erfolgen. So lassen sich auf eine schonende Weise neben einer hohen Selektivität höhere Umsätze und deutlich weniger Nebenprodukte erhalten.

In einer weiteren Ausgestaltung des erfindungsgemäßen Verfahrens ist es bevorzugt, dass die die Dehydratisierung zu mindestens teilweise oder auch vollständig in einer Gasphase erfolgt. Die Dehydratisierung in Gasphase hat sich insbesondere bei der Umsetzung von Glycerin aus der Fettverseifung wie bei der Biodieselherstellung bewährt. Dieses Glycerin führt in der Regel eine hohe Salzfracht mit sich, die durch den Verdampfungsschritt der Gasphasendehydratisierung recht gut abgetrennt werden kann. Wie auch bei der Flüssigphasendehydratisierung ist es auch bei der Gasphasendehydratisierung bevorzugt, dass diese in Gegenwart von Wasser erfolgt.

Folglich ist es in dem erfindungsgemäßen Verfahren bevorzugt, dass das Glycerin in einer wässrigen Phase eingesetzt wird. Im Fall der Flüssigphasendehydratisierung weist diese flüssige Glycerinphase im allgemeinen einen Wassergehalt in einem Bereich von 0 bis 97 Gew.-%, beispielsweise 0 bis 30 Gew. %, vorzugsweise in einem Bereich von 60 bis 95 Gew.-%, besonders bevorzugt in einem Bereich von 70 bis 90 Gew.-% auf, wobei jedoch auch ein kleinerer Wassergehalt, beispielsweise ein Wassergehalt in einem Bereich von 0 bis 20 Gew. % oder von 0 bis 10 Gew. % Wasser, jeweils bezogen auf die wässrige Phase, denkbar ist. Im Fall der Gasphasendehydratisierung weist die wässrige Glycerinphase im Allgemeinen eine Wassermenge im Bereich von 0 bis 97 Gew.-%, vorzugsweise in einem Bereich von 60 bis 95 Gew. % und darüber hinaus bevorzugt in einem Bereich von 70 bis 90 Gew. %, jeweils bezogen auf die wässrige Glycerinphase, auf, wobei es hier insbesondere auch vorteilhaft sein kann, kleinere Wassermengen, beispielsweise einen Wassergehalt in einem Bereich von 0 bis 20 Gew. % oder etwa von 0 bis 10 Gew. % einzusetzen. Der weitere Hauptbestandteil der Glycerinphase ist Glycerin. Weitere Vorteile der Gasphasendehydratisierung sind hohe Umsätze bis zu einem Totalumsatz mit gleichfalls hohen Raum-Zeit-Ausbeuten.

Nach einer anderen Ausgestaltung des erfindungsgemäßen Verfahrens ist es bevorzugt, Gasphasendehydratisierung und Flüssigphasendehydratisierung miteinander zu kombinieren. Nach einer Form des erfindungsgemäßen Verfahrens kann das Glycerin zunächst der Gasphasendehydratisierung und anschließend der Flüssigphasendehydratisierung oder in umgekehrter Reihenfolge zugeführt werden. Bei der erstgenannten Abfolge besteht der Vorteil, dass aus der Fettverseifung stammende, stark mit Salz befrachtete Glycerinchargen zunächst durch eine Verdampfung in der Gasphasendehydratisierung von dieser Salzfracht bereit werden können um dann anschließend in der Flüssigphasendehydratisierung durch die Kreisfahrweise weiter zu hohen Ausbeuten und Selektivitäten mit wenigen Nebenprodukten umgesetzt werden.

Nach einer weiteren Ausgestaltung des erfindungsgemäßen Verfahrens wird bei diesem ein Dehydratisierungskatalysator eingesetzt. Als Dehydratisierungskatalysatoren kommen sowohl saure als auch alkalische Katalysatoren in Betracht. Saure Katalysatoren sind insbesondere wegen der geringen Neigung zur Oligomerenbildung bevorzugt. Der Dehydratisierungskatalysator kann sowohl als homogener als auch als heterogener Katalysator eingesetzt werden. Wenn der Dehydratisierungskatalysator als heterogener Katalysator vorliegt, ist es bevorzugt, dass der Dehydratisierungskatalysator mit einem Träger x. in Kontakt steht. Als Träger x. kommen alle dem Fachmann als geeignet erscheinenden Feststoffe in Betracht. In diesem Zusammenhang ist es bevorzugt, dass diese Feststoffe geeignete Porenvolumina aufweisen, die zur guten Anbindung und Aufnahme des Dehydratisierungskatalysators geeignet sind. Außerdem sind Gesamtporenvolumen nach DIN 66133 in einem Bereich von 0,01 bis 3 ml/g bevorzugt und in einem Bereich von 0,1 bis 1,5 ml/g besonders bevorzugt. Zudem ist es bevorzugt, dass die als Träger x. geeigneten Feststoffe eine Oberfläche im Bereich von 0,001 bis 1000 m²/g, vorzugsweise im Bereich von 0,005 bis 450 m²/g und darüber hinaus bevorzugt im Bereich von 0,01 bis 300 m²/g nach BET-Test gemäß DIN 66131 aufweisen. Als Träger für den Dehydratisierungskatalysator kann zum einen ein Schüttgut, das einen mittleren Teilchendurchmesser im Bereich von 0,1 bis 40 mm, vorzugsweise im Bereich von 1 bis 10 mm und darüber hinaus bevorzugt im Bereich von 1,5 bis 5 mm aufweist, eingesetzt werden. Ferner kann die Wand des Dehydratisierungsreaktors als Träger dienen. Weiterhin kann der Träger an sich sauer oder basisch sein oder ein saurer oder basischer Dehydratisierungskatalysator auf einen inerten Träger aufgebracht werden. Als Aufbringtechniken sind insbesondere Eintauchen bzw. Imprägnieren oder das Einarbeiten in eine Trägermatrix zu nennen.

Als Träger x., die auch Dehydratisierungskatalysatoreigenschaften aufweisen können, eignen sich insbesondere natürliche oder synthetische silikatische Stoffe, wie insbesondere Mordenit, Montmorillonit, saure Zeolithe; mit ein-, zwei oder mehrbasigen anorganischen Säuren, insbesondere mit Phosphorsäure beaufschlagt, oder saure Salze anorganischer Säuren belegte Trägerstoffe, wie oxidische oder silikatische Stoffe, beispielsweise Al₂O₃, TiO₂; Oxide und Mischoxide, wie beispielsweise gamma-Al₂O₃ und ZnO-Al₂O₃- sowie Cu-Al-Mischoxide der Heteropolysäuren.

Es entspricht einer erfindungsgemäßen Ausführungsform, dass der Träger x. mindestens teilweise aus einer oxidischen Verbindung besteht. Derartige oxidische Verbindungen sollten mindestens eines der Elemente aus Si, Ti, Zr, Al, P oder eine Kombination von mindestens zwei davon aufweisen. Derartige Träger können auch selber durch ihre sauren bzw. basischen Eigenschaften als Dehydratisierungskatalysator wirken. Eine bevorzugte sowohl als Träger als x. als auch als Dehydratisierungskatalysator wirkende Verbindungsklasse beinhalten Silicium-, Aluminium- und/oder Phosphoroxide. Bevorzugte basische sowohl als Dehydratisierungskatalysator als auch als Träger x. fungierende Stoffe beinhalten Alkali, Erdalkali, Lanthan, Lanthanoide oder eine Kombination von mindestens zwei davon in ihrer oxidischen Form. Derartige saure oder basische Dehydratisierungskatalysatoren sind sowohl bei der Degussa AG als auch bei der Südchemie AG kommerziell erhältlich. Eine weitere Klasse stellen Ionenaustauscher dar. Auch diese können sowohl in basischer als auch in saurer Form vorliegen.

Als homogene Dehydratisierungskatalysatoren kommen insbesondere anorganische Säuren, vorzugsweise Phosphor beinhaltende Säuren und darüber hinaus bevorzugt Phosphorsäure in Betracht. Diese anorganischen Säuren können auf dem Träger x. durch Eintauchen bzw. Imprägnieren immobilisiert werden.

Insbesondere bei der Gasphasendehydratisierung hat sich der Einsatz von heterogenen Katalysatoren besonders bewährt. Bei der Flüssigphasendehydratisierung werden jedoch sowohl homogene als auch heterogene Dehydratisierungskatalysatoren eingesetzt.

In einer weiteren Ausgestaltung des erfindungsgemäßen Verfahrens ist es bevorzugt, dass der Dehydratisierungskatalysator eine anorganische Säure ist. Unter dem Begriff Säure werden in diesem Text die Stoffe verstanden, die sich gemäß der Definition von Brönsted als Säure verhalten. Hier kommen alle dem Fachmann bekannten und geeignet erscheinenden Säuren in Betracht. Vorzugsweise sind S- und P-haltig Säuren zu nennen, wobei die P-haltigen Säuren bevorzugt sind. Im Zusammenhang mit der Flüssigphasendehydratisierung ist es weiterhin bevorzugt, dass dieser auch als homogen zu bezeichnende Dehydratisierungskatalysator in einer Lösung vorliegt. Vorzugsweise handelt es sich bei dieser Lösung um die zur Dehydratisierung eingesetzte wässrige Glycerin-haltige Phase. Diese homogenen Katalysatoren werden in einer Menge in einem Bereich von 0,0001 bis 20 Gew.-%, vorzugsweise in einem Bereich von 0,001 bis 15 Gew.-% und darüber hinaus bevorzugt in einem Bereich von 0,01 bis 10 Gew.-%, jeweils bezogen auf die zur dehydratisierende Phase, eingesetzt.

Zudem ist es bevorzugt, dass in dem erfindungsgemäßen Verfahren ein Dehydratisierungskatalysator mit einem H₀-Wert im Bereich von +1 bis -10, vorzugsweise in einem Bereich von +2 bis -8,2 und darüber hinaus bevorzugt bei der Flüssigphasendehydratisierung in einem Bereich von +2 bis -3 und in der Gasphasendehydratisierung in einem Bereich von -3 bis -8,2 eingesetzt wird. Der H₀-Wert entspricht der Säurefunktion nach Hammett und lässt sich durch die sogenannte Amintritation und Verwendung von Indikatoren oder durch Absorption einer gasförmigen Base ermitteln - siehe *"*Studies in Surface Science and Catalytics", vol. 51, 1989: "New solid Acids and Bases, their catalytic Properties", K. Tannabe et al. Weitere Einzelheiten zur Herstellung von Acrolein aus Glycerin sind weiterhin DE 42 38 493 C1 zu entnehmen.

In einer weiteren Ausgestaltung des erfindungsgemäßen Verfahrens erfolgt die Gasphasenoxidation im Schritt b) des erfindungsgemäßen Verfahrens in Gegenwart eines oder mehrerer Oxidationskatalysatoren, welche Übergangsmetalle in elementarer oder in chemisch gebundener Form oder beides aufweisen. Bei den Oxidationskatalysatoren ist es bevorzugt, dass diese wenigstens eines der Elemente Molybdän, Wolfram, Vanadium oder eine Kombination aus mindestens zwei davon in mindestens teiloxidischer Form aufweisen. Derartige Oxidationskatalysatoren werden vorzugsweise als heterogene Katalysatoren mit einem Träger y. in Kontakt stehend eingesetzt. Hierbei ist es bevorzugt, dass die Oxidationskatalysatoren in diesen Träger y. eingearbeitet sind. Als geeignete Träger y. kommen grundsätzlich die im Zusammenhang mit den Trägern x. genannten Verbindungen in Betracht, wobei Träger auf Siliciumoxid - oder Aluminiumoxid- oder Aluminiumsiliciumoxid-Basis besonders bevorzugt sind. Derartige Oxidationskatalysatoren sind in der Literatur eingehend beschrieben. Hierzu wird beispielhaft auf DE-A-26 26 887, EP-A-0 534 294 sowie auf US-A-2002/0198406 verwiesen. Derartige Oxidationskatalysatoren für die Umsetzung von Acrolein zu Acrylsäure sind beispielsweise von der Mitsubishi KK kommerziell erhältlich.

Weiterhin ist es in dem erfindungsgemäßen Verfahren bevorzugt, dass das Dehydratisierungsprodukt, gegebenenfalls in einer wässrigen Phase, der Gasphasenoxidation zugeführt wird. Hierbei ist es bevorzugt, dass das Dehydratisierungsprodukt mindestens 10 Gew. %, bevorzugt mindestens 20 Gew. % und darüber hinaus bevorzugt mindestens 40 Gew. % Acrolein aufweist. Die Wassermenge sollte im Bereich von 0,001 bis 50 Gew. %, vorzugsweise im Bereich von 0,1 bis 50 Gew.-%, besonders bevorzugt im Bereich von 10 bis 40 Gew. % und darüber hinaus bevorzugt im Bereich von 12 bis 20 Gew. % liegen, wobei sich diese und die vorstehenden Gew. %-Angaben jeweils auf die in die Gasphasenoxidation eingespeiste Phase beziehen.

Eine weitere Ausgestaltung des erfindungsgemäßen Verfahrens sieht vor, dass Glycerin als wässrige Glycerinphase mit einer Glycerinkonzentration in einem Bereich von 0,1 bis 90, vorzugsweise in einem Bereich von 1 bis 80 und besonders bevorzugt in einem Bereich von 2 bis 50 Gew.-%, jeweils bezogen auf die wässrige Glycerinphase, in einen Dehydratisierungsraum eingetragen wird. Dieser Dehydratisierungsraum beinhaltet sowohl eine Flüssigphase als auch eine Gasphase, wobei die Konzentration an Glycerin in der Flüssigphase größer ist als in der Gasphase und wobei die Konzentration an Acrolein in der Gasphase größer ist als in der Flüssigphase. Vorzugsweise ist die Konzentration an Glycerin in der Flüssigphase mindestens um den Faktor 1,1, vorzugsweise mindestens um den Faktor 2 und besonders bevorzugt mindestens um den Faktor 5 höher als in der Gasphase. Weiterhin ist es bevorzugt, dass die Konzentration des Acroleins in der Gasphase mindestens um den Faktor 1,1, vorzugsweise mindestens um den Faktor 2 und besonders bevorzugt um den Faktor 5 höher ist als in der Flüssigphase. In dieser Ausgestaltung ist es weiterhin bevorzugt, dass das in der Gasphase enthaltene Acrolein aus dem vorzugsweise als Druckreaktor ausgestalteten Dehydratisierungsraum entfernt wird. Im Zusammenhang mit dieser Ausgestaltung ist es weiterhin bevorzugt, dass mehr Dehydratisierung von Glycerin zu Acrolein in der Flüssigphase als in der Gasphase erfolgt. Vorzugsweise erfolgt in der Flüssigphase mindestens um den Faktor 1,1, besonders bevorzugt mindestens um den Faktor 2 sowie darüber hinaus bevorzugt mindestens um den Faktor 5 mehr Dehydratisierung von Glycerin zu Acrolein als in der Gasphase. Es ist in dieser Ausgestaltung ferner bevorzugt, dass das in der Flüssigphase gebildete Acrolein in die Gasphase überführt wird. Es ist bei dieser Ausgestaltung ferner bevorzugt, dass der durch die Flüssigphase eingenommene Volumenanteil des Dehydratisierungsraums größer ist als der durch die Gasphase eingenommene Volumenanteil. Der durch die Flüssigphase eingenommene Volumenanteil ist vorzugsweise mindestens um den Faktor 1,1, bevorzugt mindestens um den Faktor 2 und besonders bevorzugt mindestens um den Faktor 5 größer als der durch die Gasphase eingenommene Volumenanteil des Dehydratisierungsraums. Die hier verwendeten Faktoren können beispielsweise auf Basis von Untersuchung der Phasengleichgewichte bestimmt werden.

Vorzugsweise umfasst diese Ausgestaltung des erfindungsgemäßen Verfahrens zur Herstellung von Acrylsäure die folgenden Verfahrensschritte:
a. Dehydratisieren von Glycerin in Form einer wässrigen Glycerinlösung zu einem Acrolein aufweisenden Dehydratisierungsprodukt in Form einer wässrigen Acroleinlösung mittels Flüssigphasendehydratisierung mit mindestens teilweises Überführen der wässrigen Acroleinlösung in die Gasphase, wobei das Gewichtsverhältnis von Acrolein zu Glycerin in der Gasphase um einen Faktor von mindestens 2, vorzugsweise mindestens 4, besonders bevorzugt mindestens 10 und am meisten bevorzugt mindestens 100 größer ist als das Gewichtsverhältnis von Acrolein zu Glycerin in der wässrigen Acroleinlösung;
b. Gasphasenoxidation des Acroleins aus der Gasphase unter Erhalt eines Acrylsäure aufweisenden Monomergases;
c. in Kontakt bringen des Monomergases mit einem Quenchmittel unter Erhalt einer Acrylsäure aufweisenden Quenchphase;
d. Aufarbeiten der Quenchphase unter Erhalt einer Acrylsäure beinhaltenden Monomerphase.

Demnach umfasst das Verfahren zur Herstellung eines Polymers bei dieser besonderen Ausführungsform des erfindungsgemäßen Verfahrens die folgenden Verfahrensschritte:
A. Dehydratisieren von Glycerin in Form einer wässrigen Glycerinlösung zu einem Acrolein aufweisenden Dehydratisierungsprodukt in Form einer wässrigen Acroleinlösung mittels Flüssigphasendehydratisierung mit mindestens teilweises Überführen der wässrigen Acroleinlösung in die Gasphase, wobei das Gewichtsverhältnis von Acrolein zu Glycerin in der Gasphase um einen Faktor von mindestens 2, vorzugsweise mindestens 4, besonders bevorzugt mindestens 10 und am meisten bevorzugt mindestens 100 größer ist als das Gewichtsverhältnis von Acrolein zu Glycerin in der wässrigen Acroleinlösung;
B. Gasphasenoxidation des Acroleins aus der Gasphase unter Erhalt eines Acrylsäure aufweisenden Monomergases;
C. in Kontakt bringen des Monomergas mit einem Quenchmittel unter Erhalt einer Acrylsäure aufweisenden Quenchphase;
D. Aufarbeiten der Quenchphase unter Erhalt einer Acrylsäure beinhaltenden Monomerphase;
E. Polymerisation der Monomerphase.

Die Gasphasenoxidation wird bevorzugt in einem Temperaturbereich von 200 bis 400°C, vorzugsweise im Bereich von 250 bis 350°C und darüber hinaus bevorzugt in einem Bereich von 280 bis 340°C durchgeführt.

Ferner ist es in dem erfindungsgemäßen Verfahren bevorzugt, dass das Monomergas die Acrylsäure in einer Menge im Bereich von 5 bis 50 Gew. %, vorzugsweise in einem Bereich von 10 bis 40 Gew. % und darüber hinaus bevorzugt in einem Bereich von 15 bis 30 Gew. %, jeweils bezogen auf das Monomergas, beinhaltet.

In einer weiteren Ausgestaltung des erfindungsgemäßen Verfahrens ist es bevorzugt, als Quenchmittel im Verfahrensschritt c) des erfindungsgemäßen Verfahrens Wasser oder eine organische Verbindung mit einem Siedepunkt im Bereich von 50 bis 250°C, vorzugsweise in einem Bereich von 70 bis 180°C und darüber hinaus bevorzugt in einem Bereich von 105 bis 150°C oder Wasser und diese organische Verbindung einzusetzen. Als derartige organische Verbindung kommen insbesondere Aromaten und darüber hinaus bevorzugt alkylierte Aromaten in Betracht. Üblicherweise wird das Quenchmittel mit dem Monomergas in einer geeigneten Kolonne, vorzugsweise im Gegenstrom, in Kontakt gebracht. Für den Fall, dass das Quenchmittel zu mindestens 50 Gew. %, vorzugsweise mindestens 70 Gew. %, aus Wasser besteht, ist es bevorzugt, dass das mit Acrylsäure befrachtete wässrige Quenchmittel in einem weiteren Schritt mit einem Trennmittel, das vorzugsweise nicht mit Wasser gut löslich ist, aufgearbeitet wird. Die acrylsäurereichste Phase wird entweder einer Destillation oder einer Kristallisation oder beidem, vorzugsweise zunächst einer Kristallisation, unterzogen. Die Kristallisation kann sowohl als Schicht- als auch als Suspensionskristallisation durchgeführt werden. Geeignete Schichtkristallisationsvorrichtungen sind kommerziell von der Sulzer AG zu erhalten. Geeignete Suspensionskristallisationsverfahren bedienen sich in der Regel eines Kristallerzeugers gefolgt von einer Waschkolonne. Derartige Vorrichtungen und Verfahren sind von der Niro Prozesstechnologie BV kommerziell zu erhalten. Als Extraktions/Trennmittel kommt insbesondere eine aromatische Verbindung, darüber hinaus bevorzugt ein Alkylaromat und weiterhin bevorzugt Toluol in Betracht. Sollte eine organische Verbindung als Trennmittel eingesetzt werden, so kann diese mit Acrylsäure befrachtete organische Verbindung ebenfalls sowohl einer Destillation als auch einer Kristallisation oder einer Kombination von beiden unterzogen werden. Eine hierfür geeignete Kristallisation ist in EP-A-1 015 410 offenbart.

Zudem ist es in dem erfindungsgemäßen Verfahren bevorzugt, dass die Quenchphase die Acrylsäure in einer Menge im Bereich von 30 bis 90 Gew. %, vorzugsweise im Bereich von 35 bis 85 Gew. %, und darüber hinaus bevorzugt in einem Bereich von 45 bis 75 Gew. %, jeweils bezogen auf die Monomerphase, beinhaltet.

In einer weiteren Ausgestaltung des erfindungsgemäßen Verfahrens ist es bevorzugt, dass die Aufarbeitung der Quenchphase bei Temperaturen unterhalb des Siedepunktes des Acrylsäure erfolgt. Eine dafür geeignete Maßnahme ist es, dass die Quenchphase durch Verwendung eines entsprechend kalten Quenchmittels bereits eine Temperatur von unter 40°C aufweist. Die so temperierte Quenchphase kann dann einer Extraktions oder Kristallisation oder beides zur Aufarbeitung zugeführt werden, wobei die Temperaturen vorzugsweise in einem Bereich von - 40 bis 40°C, vorzugsweise in einem Bereich von -20 bis 39°C und besonders bevorzugt in einem Bereich von -10 bis 35°C liegen.

Nach einer weiteren Ausgestaltung des erfindungsgemäßen Verfahrens ist es bevorzugt, dass die Monomerphase die Acrylsäure in einer Menge im Bereich von 99 bis 99,98 Gew. %, jeweils bezogen auf die Monomerphase, beinhaltet. Derartige Acrylsäurengehalte in einer Monomerphase treten insbesondere auf, wenn die Aufarbeitung auf destillativem Wege erfolgt. Für den Fall, dass die Aufarbeitung auf dem Weg Extraktion und Kristallisation erfolgt, kann es bevorzugt sein, dass die Acrylsäure in einer Menge von 30 bis 70 Gew. %, vorzugsweise in einer Menge im Bereich von 40 bis 60 Gew. % und darüber hinaus bevorzugt in einer Menge in einem Bereich von 45 bis 65 Gew. % in der Monomerphase neben Wasser vorliegen und die von Wasser und Acrylsäure verschiedenen Verunreinigungen weniger als 0,02 Gew. %, bezogen auf die Monomerphase, betragen. Diese wässrige Monomerphase hat den Vorteil, dass diese ohne weiteren Verdünnungsschritt, die bei der hochkonzentrierten Monomerphase notwendig ist, in die wässrige Polymerisation der Monomerphase eingesetzt werden kann.

Zudem betrifft die Erfindung eine Vorrichtung zur Herstellung von Acrylsäure, die fluidleitend miteinander verbunden folgende Komponenten aufweist:
1a. einen Dehydratisierungsreaktor;
2a. einen Gasphasenoxidationsreaktor;
3a. eine Quencheinheit;
4a. eine Aufarbeitungseinheit.

Zudem betrifft die Erfindung eine Vorrichtung zur Herstellung von Polymeren, die fluidleitend miteinander verbunden zunächst die vorstehend aufgeführten Komponenten 1a. bis 4a. und darüber hinaus eine Polymerisationseinheit 5b. aufweist.

Unter fluidleitend wird eine Verbindung der einzelnen Komponenten oder ihrer Bestandteile durch Rohrleitungssysteme oder andere Transportmöglichkeiten für Gase und Flüssigkeiten, wie Tankwagen, verstanden.

Bei der erfindungsgemäßen Vorrichtung ist es bevorzugt, dass der Dehydratisierungsreaktor einen zur Aufnahme von Glycerin geeigneten Eduktbehälter, gefolgt von einem einen zur Aufnahme von Katalysator ausgestalteten Reaktionsbereich, wiederum gefolgt von einem Quencher mit einer Ableitung zu dem Gasphasenoxidationsreaktor aufweist. Diese Komponenten sind aus üblichen, für die chemische Industrie verwendeten, im Zusammenhang mit den Reaktionsbedingungen inerten Materialien, wie Edelstahl oder Glas ausgebildet. Für den Fall, dass der Reaktionsbereich den Katalysator als Schüttgut aufnimmt, weist dieser entsprechende Behälter auf. In einer anderen Ausgestaltung kann der Reaktionsbereich auch Wandungen aufweisen, die als Katalysator fungieren. Der Quencher ist als Kolonne ausgestaltet, in die Wasser oder hochsiedende organische Lösungsmitte Eingespeist werden kann.

Eine Weiterbildung der erfindungsgemäßen Vorrichtung weist nach dem Eduktbehälter und vor dem Reaktionsbereich einen Verdampfer auf. Diese Ausgestaltungen eignen sich insbesondere für die Gasphasendehydratisierung. Für den Fall, dass das Glycerin aus der Fettsäureverseifung mit einer hohen Salzfracht eingesetzt wird, ist es bevorzugt, dass der Verdampfer einen Salzabscheider aufweist.

Nach einer anderen Ausgestaltung der erfindungsgemäßen Vorrichtung ist es bevorzugt, dass der Reaktionsbereich einen unteren Produktauslass aufweist, der in den Quencher mündet. Diese Konstruktionsweise hat sich insbesondere im Zusammenhang mit der Gasphasendehydratisierung bewährt. Nach einer anderen Weiterbildung der erfindungsgemäßen Vorrichtung ist es bevorzugt, dass der Reaktionsbereich einen oberen Produktauslass aufweist, der in den Quencher mündet. Diese Konstruktionsweise hat sich vor allem bei der Flüssigphasendehydratisierung bewährt. In einer weiteren Ausgestaltung der erfindungsgemäßen Vorrichtung ist es bevorzugt, dass der Reaktionsbereich in einem Kreislauf eingegliedert, ist, mit denen Edukte und Reaktionsprodukt im Kreis geführt werden können.

Als Gasphasenoxidationsreaktoren kommen alle dem Fachmann geläufigen und für das erfindungsgemäße Verfahren geeignet erscheinenden Reaktoren in Betracht, die in der Lage sind, Acrolein durch Gasphasenoxidation zu Acrylsäure umzusetzen. In diesem Zusammenhang bevorzugt sind Rohrbündelreaktoren oder Plattenreaktoren, die mit einem Kühlmittel, vorzugsweise einer Salzschmelze, gekühlt werden. Diese Rohrbündel- oder Plattenreaktoren nehmen auf der dem Kühlmittel abgewandten Seite einen geeigneten Katalysator auf. Dieser kann zum einen als Pulverschüttung vorliegen und zum anderen können die Flächen der Rohre bzw. der Platten mit dem Katalysator beschichtet sein.

Als Quencheinheiten werden ebenso die bei der bisherigen großtechnischen Gasphasenoxidation von Acrolein zu Acrylsäure gängigen Typen bevorzugt eingesetzt. Derartige Quencheinheiten sind als Kolonnen oder Türme ausgebildet und können genauso wie die Reaktoren beispielsweise von der Deggendorfer Werft GmbH kommerziell erworben werden. Als Aufarbeitungseinheit kommen ebenso alle dem Fachmann aus der großtechnischen über Gasphasenoxidation von Acrolein laufenden Acrylsäuresynthese bekannten Destillations- und Kristallisationssowie Extraktionseinrichtungen in Betracht.

Als Polymerisationseinheiten, welche im Verfahrensschritt E. zur Polymerisation der Monomerphase eingesetzt wird, sind zum einen diskontinuierlich arbeitende Rührkessel und zum anderen kontinuierlich arbeitende Systeme, wie Bandpolymerisationsvorrichtungen, Extruder und dergleichen geeignet. Auf diese Polymerisationsreaktoren folgt eine Zerkleinerung und Trocknung. Der so erhaltene Superabsorberprecursor kann weiterhin einer Oberflächen- oder Nachvernetzung unterzogen werden. Hierzu finden sich nähere Angaben in dem eingangs erwähnten Werk von Graham & Buchholz. Wenn es sich bei den Polymeren um vernetzte, teilneutralisierte Polyacrylate handelt, so wird hinsichtlich der genauen Vorgehensweise auf das 3. Kapitel (Seite 69ff) in "Modern Superabsorbent Polymer Technology", F. L. Buchholz und A. T. Graham (Herausgeber) in, Wiley-VCH, New York, 1998 verwiesen, das einen Teil dieser Offenbarung bildet.

Zudem ist es bevorzugt, dass das erfindungsgemäße Verfahren zur Herstellung von Acrylsäure bzw. das erfindungsgemäße Verfahren zur Herstellung eines Polymers unter Einsatz der vorstehend beschriebenen und in den Zeichnungen näher erläuterten Vorrichtungen erfolgt.

Auf diese Art und Weise lassen sich wasserabsorbierende Polymergebilde als besonders geeignete Superabsorber erhalten.

Offenbart werden auch wasserabsorbierende Polymergebilde, erhältlich durch radikalische Polymerisation der durch das vorstehend beschriebene Syntheseverfahren erhältlichen Acrylsäure in Gegenwart von Vernetzern.

Offenbart werden auch wasserabsorbierende Polymergebilde, welche zu mindestens 25 Gew.-%, vorzugsweise zu mindestens 50 Gew.-%, noch mehr bevorzugt zu mindestens 75 Gew.-% und am meisten bevorzugt zu mindestens 95 Gew.-%, auf Acrylsäure basieren, wobei mindestens 80 Gew.-%, vorzugsweise mindestens 90 Gew.-% und am meisten bevorzugt mindestens 95 Gew.-% der zur Herstellung der wasserabsorbierenden Polymergebilde eingesetzten Acrylsäuremonomere durch ein Syntheseverfahren erhalten wurden, welches von nichtfossilischem, nachwachsendem organischen Material ausgeht. Bei diesen nichtfossilischen, nachwachsenden organischen Materialien handelt es sich insbesondere nicht um aus Erdöl, oder Stein- bzw. Braunkohle sowie Erdgas gewonnnen Materialien. Vielmehr sind diese nichtfossilischen, nachwachsenden organischen Materialien Erzeugnisse der Land- und Forstwirtschaft, insbesondere Fette und Öle aus Glycerin und Fettsäuren.

Vorzugsweise sind diese wasserabsorbierenden Polymergebilde erhältlich durch ein Verfahren umfassend die folgenden Verfahrensschritte:
i) Polymerisation der Acrylsäure in Gegenwart eines Vernetzers unter Ausbildung eines Polymergels;
ii) gegebenenfalls Zerkleinerung des Polymergels;
iii) Trocknung des gegebenenfalls zerkleinerten Polymergels unter Erhalt wasserabsorbierender Polymergebilde, sowie
iv) gegebenenfalls Oberflächennachbehandlung der wasserabsorbierenden Polymergebilde.

Gemäß einer besonderen Ausführungsform der wasserabsorbierende Polymergebilde basieren diese zu mindestens 25 Gew.-%, vorzugsweise zu mindestens 35 Gew.-% und am meisten bevorzugt zu mindestens 45 Gew.-% auf natürlichen, biologisch abbaubaren Polymeren, vorzugsweise auf Kohlehydraten wie etwa Zellulose oder Stärke.

Bevorzugte Polymergebilde sind Fasern, Schäume oder Teilchen, wobei Fasern und Teilchen bevorzugt und Teilchen besonders bevorzugt sind.

Bevorzugte Polymerfasern sind so dimensioniert, dass sie in oder als Garne für Textilien und auch direkt in Textilien eingearbeitet werden können. Es ist bevorzugt, dass die Polymerfasern eine Länge im Bereich von 1 bis 500, bevorzugt 2 bis 500 und besonders bevorzugt 5 bis 100 mm und einen Durchmesser im Bereich von 1 bis 200, bevorzugt 3 bis 100 und besonders bevorzugt 5 bis 60 Denier besitzen.

Bevorzugte Polymerteilchen sind so dimensioniert, dass sie eine mittlere Teilchengröße gemäß ERT 420.2-02 im Bereich von 10 bis 3000 µm, vorzugsweise 20 bis 2000 µm und besonders bevorzugt 150 bis 850 µm aufweisen. Weiterhin ist es bevorzugt, dass der Anteil an Partikeln mit einer Partikelgröße in einem Bereich von 300 bis 600 µm mindestens 50 Gew.-%, besonders bevorzugt mindestens 75 Gew.-% beträgt.

Weiterhin ist es bevorzugt, dass die wasserabsorbierenden Polymergebilde mindestens eine, vorzugsweise beide, der folgenden Eigenschaften aufweisen:
- einen gemäß ERT 441.2-02 (ERT = Edana Recommended Test Method) bestimmten CRC-Wert (CRC = Centrifugation Retention Capacity) von mindestens 20 g/g, vorzugsweise mindestens 25 g/g und am meisten bevorzugt mindestens 30 g/g;
- eine gemäß ERT 442.2-02 bestimmte Absorption unter einem Druck von 20 g/cm² von mindestens 15 g/g, vorzugsweise mindestens 17,5 g/g und am meisten bevorzugt mindestens 20 g/g.

Die CRC-Werte und die Werte für die Absorption unter Druck liegen im Allgemeinen nicht über 150 g/g

Weiterhin offenbart werden wasserabsorbierende Polymergebilde, welche durch folgende Eigenschaften gekennzeichnet sind:
(β1) das Polymergebilde basiert zu mindestens 25 Gew.-%, vorzugsweise zu mindestens 50 Gew.-%, noch mehr bevorzugt zu mindestens 75 Gew.-% und am meisten bevorzugt zu mindestens 95 Gew.-%, auf Acrylsäure, wobei mindestens 80 Gew.-%, vorzugsweise mindestens 90 Gew.-% und am meisten bevorzugt mindestens 95 Gew.-% der zur Herstellung der wasserabsorbierenden Polymergebilde eingesetzten Acrylsäuremonomere durch ein Syntheseverfahren erhalten wurden, welches von nichtfossilen, nachwachsendem organischen Material ausgeht;
(β2) das Polymergebilde weist eine gemäß dem modifizierten Sturm-Test gemäß Anhang V zur Richtlinie 67/548/EWG bestimmte biologische Abbaubarkeit nach 28 Tagen von mindestens 25 %, vorzugsweise mindestens 35 % und am meisten bevorzugt mindestens 45 % auf, wobei ein Wert von maximal 75 bis 95 % als Obergrenze im allgemeinen nicht überschritten wird;
(β3) das Polymergebilde weist einen gemäß ERT 441.2-02 bestimmten CRC-Wert von mindestens 20 g/g, vorzugsweise mindestens 25 g/g und am meisten bevorzugt mindestens 29 g/g auf, wobei ein CRC-Wert von 60 g/g, als Obergrenze im allgemeinen nicht überschritten wird.

In einer anderen Ausgestaltung des im vorstehenden Abschnitt beschriebenen Polymergebildes weist dieses mindestens die Eigenschaften β1 und β2 auf. Alle in diesem Text für die Polymergebilde angegebenen Weiterbildungen gelten auch für das Polymergebilde dieses Absatzes.

Ebenfalls offenbart werden wasserabsorbierende Polymergebilde, welche zu mindestens 10, vorzugsweise mindestens 25, besonders bevorzugt mindestens 50 und darüber hinaus bevorzugt mindestens 75 sowie ferner bevorzugt mindestens 80 Gew.-%, jeweils bezogen auf das Polymergebilde, auf Acrylsäure basieren, welche durch folgende Eigenschaften gekennzeichnet sind:
(ε1) das Polymergebilde zeigt einen Nachhaltigkeitsfaktor von mindestens 10, vorzugsweise mindestens 20, besonders bevorzugt mindestens 50, darüber hinaus bevorzugt mindestens 75, ferner bevorzugt mindestens 85 und weiterhin bevorzugt mindestens 95;
(ε2) das Polymergebilde weist eine gemäß dem modifizierten Sturm-Test gemäß Anhang V zur Richtlinie 67/548/EWG bestimmte biologische Abbaubarkeit nach 28 Tagen von mindestens 25 %, vorzugsweise mindestens 35 % und am meisten bevorzugt mindestens 45 % auf, wobei ein Wert von maximal 75 bis 95 % als Obergrenze im allgemeinen nicht überschritten wird;
(ε3) das Polymergebilde weist einen gemäß ERT 441.2-02 bestimmten CRC-Wert von mindestens 20 g/g, vorzugsweise mindestens 25 g/g und am meisten bevorzugt mindestens 29 g/g auf, wobei ein CRC-Wert von 60 g/g, als Obergrenze im allgemeinen nicht überschritten wird.

In einer weiteren Ausgestaltung des im vorstehenden Abschnitt beschriebenen Polymergebildes weist dieses mindestens die Eigenschaften ε1 und ε2 auf. Alle in diesem Text für die Polymergebilde angegebenen Weiterbildungen gelten auch für das Polymergebilde dieses Absatzes.

In manchen Fällen können die vorstehend genannten Obergrenzen auch bis zu 10% oder auch bis zu 20% niedriger liegen. Bei den in den beiden vorstehenden Absätzen beschriebenen Polymergebilden ist es bevorzugt, dass diese neben der Acrylsäure weiterhin auf einem Zwei- oder Mehrfachzucker basieren. Diese Zwei- oder Mehrfachzucker liegen vorzugsweise in einer Menge von mindestens 1 Gew.-%, vorzugsweise mindestens 5 Gew.-% und darüber hinaus bevorzugt mindestens 15 Gew.-%, jeweils bezogen auf das Polymergebilde, als ein weitere Bestandteil des Polymergebildes vor, so dass die Summe der Gew.-% der Bestandteile des wasserabsorbierenden Polymergebildes 100 Gew.-% ergibt. Bei derartigen Zuckern sind Kettenmehrfachzucker bevorzugt, die vorzugsweise ein mittels Gelpermeationschromatographie und Lichtstreuung ermitteltes Zahlenmittel des Molekulargewichts im Bereich von 10.000 bis 1.000.000 und vorzugsweise im Bereich von 50.000 bis 500.000 g/mol aufweisen. Diese bestehen vorzugsweise aus linearen und damit unverzeigten Ketten. Als derartige Zucker kommen alle dem Fachmann bekannten und als geeignet erscheinenden Zuckerverbindungen in betracht. So sind beispielsweise Zellulose und Stärke zu nennen, wobei ein oder mindestens zwei verschiedenen Stärken bevorzugt sind. Unter den Stärken sind wiederum amylosehaltige Stärken bevorzugt. Der Amylosegehalt liegt vorzugsweise in einem Bereich von 10 bis 80 und besonders bevorzugt in einem Bereich von 20 bis 70 Gew.-%, jeweils bezogen auf die Stärke. Weiterhin ist es bevorzugt, dass die Zwei- oder Mehrfachzucker eine Teilchengröße aufweisen, bei der mindestens 50 Gew.-%, vorzugsweise mindestens 70 Gew.-% und darüber hinaus bevorzugt mindestens 85 Gew.-% der Teilchen kleiner 50µm sind. Die Teilchengröße wird durch Siebanalyse bestimmt. Derartige Produkte sind beispielsweise unter der Marke Eurylon^{®} 7 oder Foralys^{®} 380 der Firma Roquette - Frankreich - kommerziell erhältlich.

Hergestellt werden können und damit erhältlich sind derartige wasserabsorbierende Polymergebilde vorzugsweise durch
- Bereistellen eines oberflächenvernetzten wasserabsorbierenden Polymers;
- Mischen des oberflächenvernetzten wasserabsorbierenden Polymergebildes mit einem Zwei- oder Mehrfachzucker.

Hierbei ist es bevorzugt, dass das wasserabsorbierende Polymer zu mindestens 50 Gew.-%, vorzugsweise mindestens 80 Gew.-% und darüber hinaus bevorzugt mindestens 95 Gew.-% auf Acrylsäure basiert, die aus dem erfindungsgemäßen Dehydratisierungsverfahren stammt und teilneutralisiert mit einem Vernetzer zur Polymerisation eingesetzt wird. Die Oberflächenvernetzung wird durch die hier bereits beschriebene Nachvernetzung erreicht.

Der Nachhaltigkeitsfaktor gibt an, zu welchem Anteil das Polymergebilde auf Stoffen basierend auf nichtfossilen, nachwachsendem organischen Material basiert. Bei einem Nachhaltigkeitsfaktor von 100 besteht das Polymergebilde vollständig aus auf nichtfossilen, nachwachsendem organischen Materialen basierenden Stoffen.

Ebenfalls offenbart wird ein Verbund, beinhaltend die wasserabsorbierenden Polymergebilde bzw. wasserabsorbierenden Polymergebilde, die durch radikalische Polymerisation der durch das vorstehend beschriebene Syntheseverfahren erhältlichen Acrylsäure in Gegenwart von Vernetzern erhältlich sind. Es ist dabei bevorzugt, dass die Polymergebilde und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es bevorzugt, dass die Polymergebilde in einer Menge von mindestens 50 Gew.-%, vorzugsweise mindestens 70 Gew.-% und noch mehr bevorzugt mindestens 90 Gew.-%, bezogen auf das Gesamtgewicht aus Polymergebilde und Substrat, in dem Verbund enthalten sind.

In einer besonders bevorzugten Ausführungsform des Verbundes handelt es sich um einen flächenförmigen Verbund, wie er in der WO-A-02/056812 als "absorbent material" beschrieben ist. Der Offenbarungsgehalt der WO-A-02/056812, insbesondere hinsichtlich des genauen Aufbaus des Verbundes, des Flächengewichtes seiner Bestandteile sowie seiner Dicke wird hiermit als Referenz eingeführt und stellt einen Teil der Offenbarung der vorliegenden Erfindung dar.

Offenbart wird auch ein Verfahren zur Herstellung eines Verbundes, wobei die wasserabsorbierenden Polymergebilde bzw. die wasserabsorbierenden Polymere, die durch radikalische Polymerisation der durch das vorstehend beschriebene Syntheseverfahren erhältlichen Acrylsäure in Gegenwart von Vernetzern erhältlich sind, und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem Verbund genannt wurden.

Offenbart wird auch ein Verbund erhältlich nach dem vorstehend beschriebenen Verfahren.

Ebenfalls offenbart werden chemische Produkte beinhaltend die wasserabsorbierenden Polymergebilde oder einen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze. Bevorzugte chemische Produkte sind Hygieneartikel, umfassend eine für Oberschicht, eine für Unterschicht sowie eine zwischen der Oberschicht und der Unterschicht angeordnete Zwischenschicht, welche wasserabsorbierende Polymergebilde beinhalten.

Zudem wird offenbart ein Verfahren zur Herstellung von Acrolein das durch das hierin beschriebene Verfahren zur Dehydratisierung von Glycerin zu einem Acrolein aufweisenden Dehydratisierungsprodukt und die hierin beschriebenen bevorzugten Ausführungsformen dieser Dehydratisierung gekennzeichnet ist.

Weiterhin offenbart werden Fasern, Folien, Klebstoffe, Kosmetika, Formmassen, Textil- und Lederhilfsmittel, Flockungsmittel, Beschichtungen oder Lacke basierend auf Acrylsäure, die nach einem erfindungsgemäßen Verfahren erhältlich ist, oder deren Derivate oder Salze. Als Derivate der Acrylsäure kommen insbesondere ihre Ester, vorzugsweise ihre Alkylester und darüber hinaus bevorzugt ihre C₁-bis C₁₀-, darüber hinaus bevorzugt C₂- bis C₅- und weiterhin bevorzugt C₃- bis C₄-Alkylester in Betracht. Als Salze sind die Alkali- oder Erdalkali- sowie die Ammoniumsalze der Acrylsäure zu nennen.

Ferner offenbart wird die Verwendung einer Acrylsäure, die durch ein erfindungsgemäßes Verfahren erhalten wurde, oder deren Derivate oder Salze in Fasern, Folien, Klebstoffen, Kosmetika, Formmassen, Textil- und Lederhilfsmitteln, Flockungsmitteln, Beschichtungen oder Lacken.

Die Erfindung wird nun anhand nicht limitierender Zeichnungen und Beispiele näher erläutert.
- Fig. 1: zeigt einen schematischen Ablauf der einzelnen Stufen und Schritte des erfindungsgemäßen Verfahrens und der erfindungsgemäßen Vorrichtung.
- Fig. 2: zeigt eine Gasphasendehydratisierungseinheit.
- Fig. 3: zeigt eine Flüssigphasendehydratisierungseinheit.
- Fig. 4: zeigt eine Aufarbeitungseinheit.
- Fig. 5: zeigt eine weitere Ausgestaltung der Flüssigphasendehydratisierungseinheit

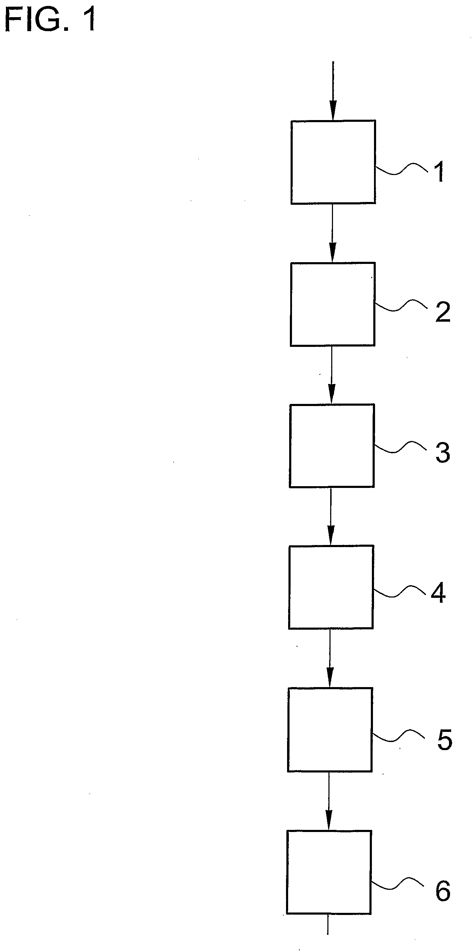

In Fig. 1 werden zunächst die Öle oder Fette in einen Verseifer 1 eingegeben, wo eine alkalische Verseifung mit Laugen oder Alkalialkoholaten erfolgt. Das in dem Verseifer nach allgemein bekannten Reinigungsschritten wie Aussalzen und Destilieren gewonnene Glycerin wird dann einer Dehydratisierungseinheit mit einem Dehydratisierungsreaktor 2 zugeführt (um aus dem Glycerin Acrolein zu gewinnen). Das so gewonnene Acrolein wird dann in einem nächsten Schritt einem Gasphasenreaktor 3 zugeführt, in dem es durch eine Gasphasenoxidationsreaktion zu Acrylsäure umgesetzt wird. Auf den Gasphasenreaktor 3 folgt eine Quencheinheit 4, in der das acrylsäurehaltige Gas aus dem Gasphasenreaktor 3 in die flüssige Phase durch in Kontakt bringen mit einem Quenchmittel gebracht wird. Die flüssige Mischung aus Quenchmittel und Acrylsäure wird einer auf die Quencheinheit 4 folgenden Aufarbeitungseinheit 5 zugeführt. Dort wird die Acrylsäure entweder durch Kristallisation oder Destillation oder einer Kombination dieser beiden Schritte oder auch durch Extraktion oder einer Kombination aus Extraktion und Kristallisation oder einer Kombination aus Extraktion und Destillation oder einer Kombination aus Extraktionsdestillation und Destillation zu reiner Acrylsäure (mindestens 99,98 % Acrylsäure) aufgereinigt, die entweder als reine Acrylsäure für sich oder in einer wässrigen Phase vorliegt. Die so gewonnene Acrylsäure wird dann einer Polymerisationseinheit 6 zugeführt. Das in der Polymerisationseinheit 6 gewonnene Polymer kann entsprechend der nachfolgenden Verwendung konfektioniert werden. Auf die Polymerisationseinheit 6 kann eine Weiterverarbeitungseinheit, beispielsweise eine Windelmaschine oder eine Maschine zur Herstellung von Verbands- und Wundmaterialien folgen.

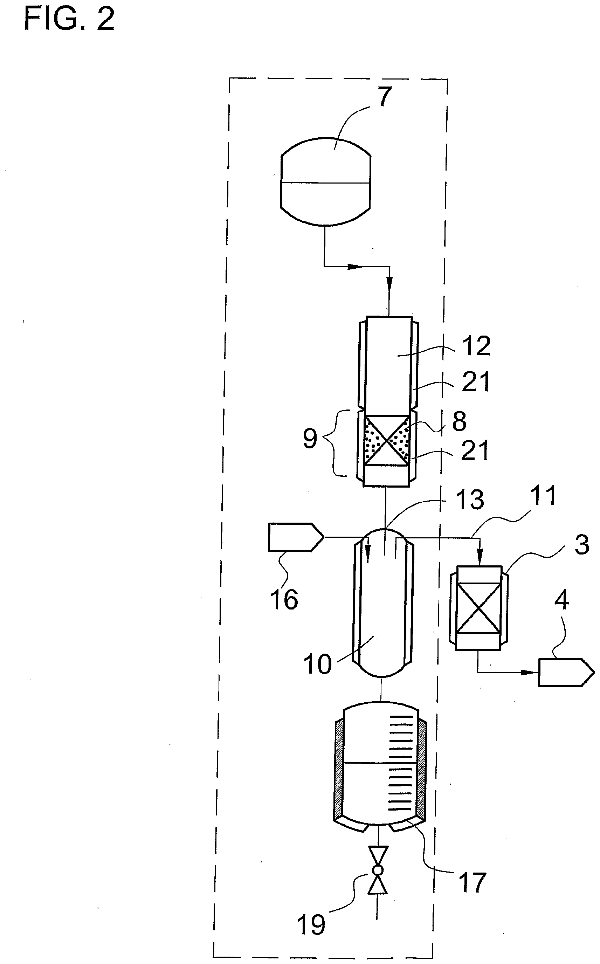

Fig. 2 zeigt eine Gasphasendehydratisierungseinheit, in der ein Eduktbehälter 7 mit einem Verdampfer 12 verbunden ist. Der Verdampfer 12 ist einem Reaktionsbereich 9 vorgeschaltet. Der druckbelastbare Reaktionsbereich 9 weist Katalysator 8 auf. Sowohl der Verdampfer 12 als auch der Reaktionsbereich 9 weisen Heizelemente 21 auf, mit denen die zur Verdampfung und Dehydratisierung des Glycerins notwendigen Temperaturen eingestellt werden können. Über einen unteren Auslass 13 ist der Reaktionsbereich 9 mit einem Quencher 10 verbunden. Der Quencher 10 nimmt in seinem oberen Bereich neben dem unteren Produktauslass 13 noch eine Quenchflüssigkeitseinspeisung 16 auf. Im unteren Bereich des Quenchers 10 schließt sich ein Auffangbehälter für Nebenprodukte 17 an, der über ein Ablassventil 19 entleerbar ist. Der obere Bereich des Quenchers 10 nimmt weiterhin eine Ableitung 11 auf, die das acroleinhaltige Dehydratisierungsprodukt dem Gasphasenreaktor 3 zuführt. Auf diesen folgt die Quencheinheit 4.

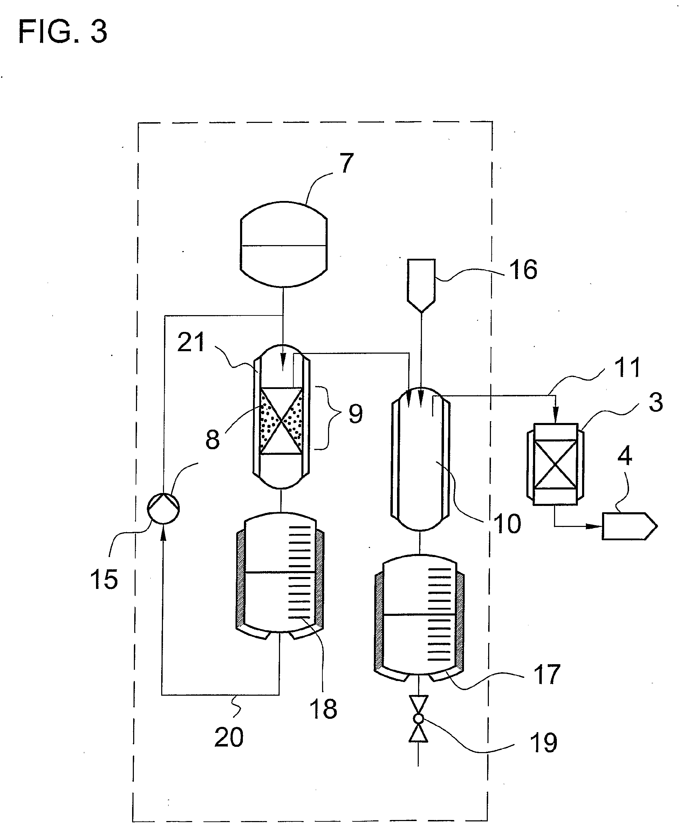

Fig. 3 zeigt eine Flüssigphasendehydratisierungsvorrichtung, in der Glycerin in einem Eduktbehälter 7 vorgelegt wird, der mit dem oberen Bereich eines druckbelastbaren Reaktionsbereichs 9 verbunden ist, der wiederum Katalysatoren 8 aufweist. Der Reaktionsbereich 9 wird durch ein Heizelement 21 temperiert. Im unteren Bereich des Reaktionsbereichs 9 schließt sich ein Pufferbehälter 18 an, der über eine Pumpe mit dem Reaktionsbereich einen Kreislauf 20 bildet, über dem bereits in dem Reaktionsbereich 9 und dem Pufferbehälter 18 befindliches Glycerin und Acrolein im Kreis geführt werden kann. Im oberen Bereich des Reaktionsbereichs 9 befindet sich eine Überleitung zu einem Quencher 10, die in den oberen Bereich des Quenchers 10 zusammen mit einer Quenchflüssigkeitseinspeisung 16 einmündet. Ebenfalls im oberen Bereich des Quenchers 10 befindet sich eine Ableitung 11 zu dem Gasphasenreaktionsreaktor 3, auf den wiederum die Quencheinheit 4 folgt. Im unteren Bereich des Reaktionsbereichs 9 mündet ein mit einem Ablassventil 19 ausgestatteter Auffangbehälter für Nebenprodukte 17.

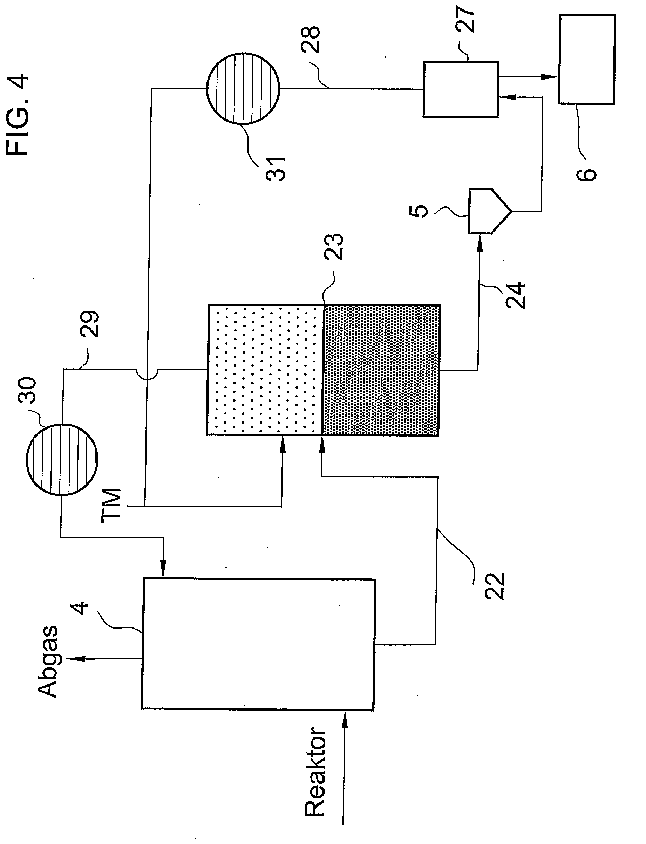

In Fig. 4 wird das aus dem Gasphasenreaktionsreaktor 3 stammende Gas in die Quencheinheit 4 eingeführt, in der es mit Wasser in Kontakt gebracht und absorbiert wird, wobei als Absorptionsflüssigkeit eine wässrige Acrylsäurelösung erhalten wird, in der ebenso ein Teil der in den vorstehenden Syntheseschritten entstandenen Nebenprodukte enthalten ist. Über eine Zuführung 22 wird diese wässrige Acrylsäurelösung einer Extraktionseinheit 23 zugeleitet. Spätestens in der Extraktionseinheit 23 wird die Acrylsäurelösung mit Toluol als Trennmittel (TM) in Kontakt gebracht. Nach einem sorgfältigen Vermischen in der Extraktionseinheit 23 erfolgt eine Phasentrennung unter Bildung einer oberen, im Wesentlichen auf Wasser basierenden Phase und einer unteren, im wesentlichen auf Toluol als Trennmittel basierenden Phase. Die im Wesentlichen auf dem Trennmittel basierenden Phase wird über die Zuführung 24 dem Kristallisator 25, bei dem es sich vorzugsweise um einen Kratzkühler handelt, zugeführt. Die im Kristallisator 25 erhaltene Kristallsuspension wird anschließend über eine Suspensionszuführung 26 einer Waschkolonne 27 zugeführt, in der eine Abrennung der Acrylsäurekristalle erfolgt, in deren Verlauf eine das Trennmittel beinhaltende Mutterlauge zurückbehalten wird. Die in der Waschkolonne 27 nach der Abtrennung der Acrylsäurekristalle erhaltene Mutterlauge wird vorzugsweise zumindest teilweise über die Mutterlaugenführung 28 in die Extraktionseinheit 23 zurückgeführt. Es ist weiterhin bevorzugt, dass die in der Extraktionseinheit 23 erhaltene, im wesentlichen auf Wasser basierende obere Phase über die Quenchführung 29 in die Quencheinheit 4 zurückgeführt wird. In einer besonderen Ausgestaltung des erfindungsgemäßen Verfahrens und der erfindungsgemäßen Vorrichtung kann dabei aus der in der Quenchführung 29 geführten Zusammensetzung mittels einer weiteren Aufreinigungsvorrichtung 30, bei der es sich beispielsweise um eine Suspensionskristallisationsvorrichtung oder einen Schichtkristaller handelt, noch in der Zusammensetzung enthaltene Acrylsäure durch Kristallisation abgetrennt werden. Ferner ist es in einer anderen Ausführungsform des erfindungsgemäßen Verfahrens und der erfindungsgemäßen Vorrichtung bevorzugt, dass die in der Mutterlaugenführung 28 geführte Zusammensetzung (bei der Kristallisation abgetrennte Mutterlauge) vor ihrer Rückführung mittels einer Trenneinheit 31 von weiteren Verunreinigungen befreit wird. Bei dieser Trenneinheit handelt es sich vorzugsweise ebenfalls um eine Suspensionskristallisationsvorrichtung oder um einen Schichtkristaller oder auch um einen Filter.

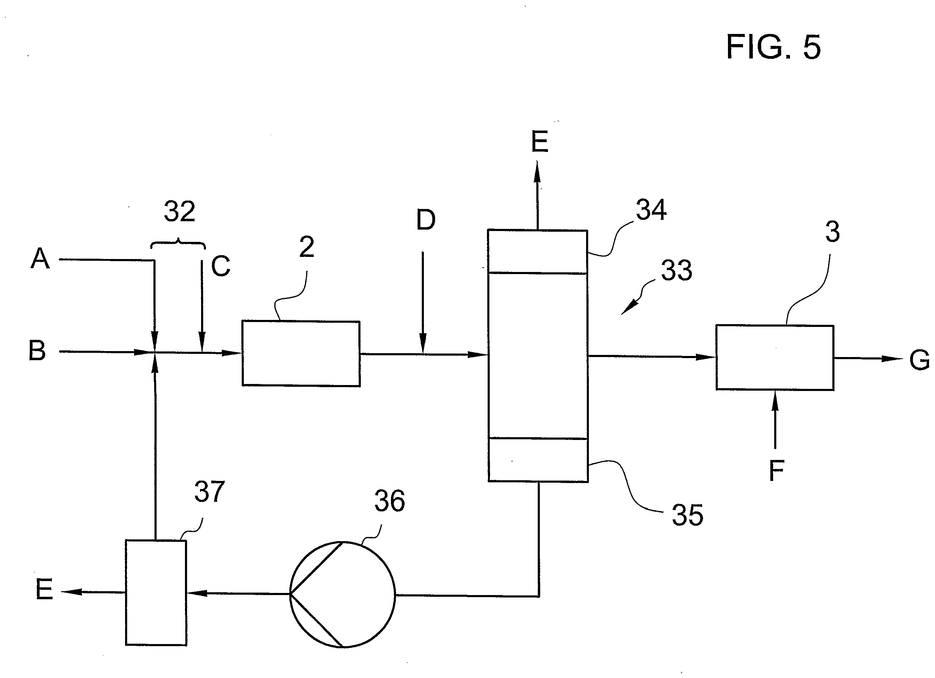

In Fig. 5 wird schematisch gezeigt, dass gegebenenfalls in einem Vormischer 32, über die Zuleitungen A bis C Wasser, Glycerin und, sofern erforderlich, Katalysator, beispielsweise eine anorganische Säure wie Phosphorsäure vermischt und dem als Edelstahldruckreaktor ausgebildeten Dehydratisierungsreaktor 2 zugeleitet werden. Hier erfolgt die Dehydratisierung von Glycerin zu Acrolein in der Flüssigphase. Bei Einsatz eines löslichen und damit homogen verteilbaren Katalysators wie einer anorganischen Säure ist es vorteilhaft diese zumindest teilweise durch die Zugabe einer Base wie NaOH, meist als Lösung, über Zuleitung D zu neutralisieren. Diese Maßnahme trägt zur Unterbindung weiterer Reaktionen, die zu unerwünschten Nebenprodukten führen können, bei. Im Anschluss daran befindet sich eine Destillationsvorrichtung 33 die im oberen Abschnitt einen Leichtsiederbereich 34 und im unteren Abschnitt einen Schwersiederbereich 35 aufweist, wobei die acroleinhaltige Phase aus der Dehydratisierung zwischen den beiden Bereichen 34 und 35 eingespeist wird. In dem Leichtsiederbereich 34 können die niedriger als Acrolein siedenden Bestandteile über Ableitung E herausgeführt und das noch verbliebene Acrolein und die Schwersieder im Rückfluss geführt. In dem den Destillensumpf darstellenden Schwersiederbereich 35 werden die Schwersieder aufkonzentriert. Hierbei ist es vorteilhaft, dass das Sumpfprodukt in einem Kreislauf gefahren wird. Aus dem Schwersiederbereich werden die höher als Acrolein siedenden Bestandteile ausgetragen und über eine Schwersiederpumpe einem Schwersiederabscheider 37 zugeführt. Der Schwersiederabscheider 37 ist vorteilhafter Weise mit einer Membran ausgestattet. Das von den Schwersiedern befreite, vornehmlich Wasser und Glycerin aufweisende Gemisch, wird nach Durchlaufen des Schwersiederabscheiders 37 wieder dem Vormischer 32 zugeführt und steht somit der Dehydratisierung wieder zur Verfügung. Durch diese Maßnahmen kann über einen langen Zeitraum eine über die Kreislauffahrweise effiziente Dehydratisierung von Glycerin zu Acrolein erreicht werden, bei der das so gewonnene Acrolein aus der Destille 33 in für einen lang andauernden Betrieb der Gasphasenoxidation ausreichenden Reinheit dem Gasphasenreaktionsreaktor 3 zur Oxidation mit durch Zuleitung F zugeführter Luft zu über Ableitung G abgeführten Acrolein zugeleitet werden.

### BEISPIELE

### Beispiel 1: Dehydratisierung in der Gasphase

In einer in Fig. 2 beschriebenen Gasphasendehydratisierungsvorrichtung (gestrichelt eingegrenzt) werden in dem Reaktorbereich 9 ein Katalysator vorgelegt, der aus 100 Gew.-Teilen Rosenthal-Kugeln (alpha-Al₂O₃) mit einem Durchmesser von 3 mm durch Mischen mit 25 Gew.-Teilen einer 20 Gew.-%igen Phosphorsäure über eine Stunde erhalten wurde und anschließend am Rotationsverdampfer bei 80°C vom überschüssigen Wasser befreit wurde (H₀-Wert zwischen -5,6 und -3). Das in einem Verdampfer bei 295°C verdampfte Glycerin wurde auf 100 ml dieses Katalysators in einem Stahlrohr in dem Reaktionsbereich als 20 Gew.-%ige wässrige Lösung mit einer Pumpe mit 40 ml/h und bei einer Temperatur von 270°C gefördert. Das acroleinhaltige Reaktionsgemisch wurde mit Wasser als Quenchmittel in dem Quencher in Kontakt gebracht und das so erhaltene wässrige Gemisch der Gasphasenoxidation in einem handelsüblichen Reaktor zur Gasphasenoxidation von Acrolein zu Acrylsäure zugeführt.

### Beispiel 2: Flüssigphasendehydratisierung

In einer Vorrichtung gemäß Fig. 3 wurde zur Flüssigphasendehydratisierung (gestrichelt eingegrenzt) ein wie in Beispiel 1 beschriebener Katalysator verwendet, wobei anstelle der Rosentahl-Kugeln auf einem Silizumdioxidträger eingesetzt wurden (H₀-Wert zwischen 2 und -3). Die Reaktionstemperatur betrug 240°C. Als Quenchmittel wurde Wasser eingesetzt. Im Anschluss an die Acroleinsynthese erfolgte ebenso wie in Beispiel 1 eine Gasphasenoxidation in einem handelsüblichen Gasphasenoxidationsreaktor, gefolgt von einer Absorption in Wasser in einer Quencheinheit.

Die in den Beispielen 1 und 2 erhaltenen Acrylsäurewassergemische wurden in einem auf 0°C temperierten Glasscheidetrichter mit 0,5 Teilen ihres Volumen mit Toluol versetzt. Die Mischung wurde kräftig geschüttelt und über 60 Minuten stehengelassen, um eine Phasentrennung zu ermöglichen. Die beiden so entstandenen Phasen wurden getrennt. Die toluolhaltige Phase wird einer azeotropen Destillation unterzogen und die dabei erhaltene Acrylsäure vor ihrem Einsatz zur Polymerisation nochmals destilliert.

Weder bei der durch Gasphasendehydratisierung noch bei der durch Flüssigphasendehydratisierung und anschließender Gasphasenoxidation des so gewonnen Acroleins erhaltenen Acrylsäure konnte PTA als Verunreinigung durch Gaschromatographie nachgewiesen werden.

### Beispiel 3: Polymerisation

Eine Monomerenlösung bestehend aus 280 g der vorstehend gewonnenen Acrylsäure, die zu 70 Mol-% mit Natronlauge neutralisiert wurde, 466,8 g Wasser, 1,4 g Polyethylenglykol-300-diacrylat und 1,68 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,1 g 2,2'-Azobis-2-amidinpropan-dihydrochlorid in 10 g H₂O, 0,3 g Natriumperoxydisulfat in 10 g H₂O, 0,07 g 30%ge Wasserstoffperoxidlösung in 1 g H₂O und 0,015 g Ascorbinsäure in 2 g H₂O zugesetzt. Nachdem die Endtemperatur von ca. 100°C erreicht war, wurde das entstandene Gel zerkleinert und bei 150°C 90 Minuten lang getrocknet. Das getrocknete Polymerisat wurde grob zerstoßen, gemahlen und auf ein Pulver mit einer Partikelgröße von 150 bis 850 µm gesiebt.

Zur Nachvernetzung wurden 100g des oben erhaltenen Pulvers unter kräftigen Rühren mit einer Lösung aus 1 g 1,3-Dioxalan-2-on, 3g Wasser und 0,5 g Aluminiumsulfat-18-Hydrat vermischt und anschließend für 40 min in einem Ofen, der auf 180°C temperiert war, erhitzt.

### Beispiel 4: Herstellung eines biologisch abbaubaren Polymers

Das im Beispiel 3 erhaltene, nachvernetzte Polymer wurde mit einer wasserlöslichen Weizenstärke (dem Produkt Foralys^{®}380 der Firma Roquette, Lestrem, Frankreich) im Gewichtsverhältnis Polymer: Stärke von 4 : 1 unter trockenen Bedingungen gemischt und anschließend für 45 Minuten in einem Rollenmixer Typ BTR 10 der Firma Fröbel GmbH, Deutschland, weiter homogenisiert.

Das Produkt wies eine biologische Abbaubarkeit gemäß dem modifizierten Sturmtest nach 28 Tagen von 39 % und einen CRC-Wert von 29,9 g/g auf. Der Nachhaltigkeitsfaktor betrug etwa 99.

### Bezugszeichenliste

- 1: Verseifer
- 2: Dehydratisierungsreaktor
- 3: Gasphasenreaktionsreaktor
- 4: Quencheinheit
- 5: Aufarbeitungseinheit
- 6: Polymerisationseinheit
- 7: Eduktbehälter
- 8: Katalysator
- 9: Reaktionsbereich
- 10: Quencher
- 11: Ableitung
- 12: Verdampfer
- 13: unterer Produktauslass
- 14: oberen Produktauslass
- 15: Pumpe
- 16: Quenchflüssigkeitseinspeisung
- 17: Auffangbehälter für Nebenprodukte
- 18: Pufferbehälter
- 19: Ablassventil
- 20: Kreislauf
- 21: Heizelement
- 22: Zuführung
- 23: Extraktionseinheit
- 24: Zuführung
- 25: Kristallisator
- 26: Suspensionsführung
- 27: Waschkolonne
- 28: Mutterlaugenführung
- 29: Quenchführung
- 30: Aufreinigungsvorrichtung
- 31: Trenneinheit
- 32: Vormischer
- 33: Destillationsvorrichtung
- 34: Leichtsiederbereich
- 35: Schwersiederbereich
- 36: Schwersiederpumpe
- 37: Schwersiederabscheider

## Patentansprüche

1. Verfahren zur Herstellung von Acrylsäure, mindestens die folgenden Schritte aufweisend:
a. Dehydratisieren von Glycerin zu einem Acrolein aufweisenden Dehydratisierungsprodukt;
b. Gasphasenoxidation des Dehydratisierungsprodukts unter Erhalt eines Acrylsäure aufweisenden Monomergas;
c. in Kontakt bringen des Monomergas mit einem Quenchmittel unter Erhalt einer Acrylsäure aufweisenden Quenchphase;
d. Aufarbeiten der Quenchphase unter Erhalt einer Acrylsäure beinhaltenden Monomerphase.

2. Verfahren zur Herstellung eines Polymers durch radikalische Polymerisation von Acrylsäure, mindestens die folgenden Schritte aufweisend:
A. Dehydratisieren von Glycerin zu einem Acrolein aufweisenden Dehydratisierungsprodukt;
B. Gasphasenoxidation des Dehydratisierungsprodukts unter Erhalt eines Acrylsäure aufweisenden Monomergas;
C. in Kontakt bringen des Monomergas mit einem Quenchmittel unter Erhalt einer Acrylsäure aufweisenden Quenchphase;
D. Aufarbeiten der Quenchphase unter Erhalt einer Acrylsäure beinhaltenden Monomerphase;
E. radikalische Polymerisation der Monomerphase.

3. Verfahren nach Anspruch 2, wobei das Polymer ein wasserabsorbierendes Polymergebilde ist.

4. Verfahren nach Anspruch 3, wobei das wasserabsorbierende Polymergebilde erhältlich ist durch ein Verfahren umfassend die folgenden Verfahrensschritte:
i) Polymerisation der Acrylsäure in Gegenwart eines Vernetzers unter Ausbildung eines Polymergels;
ii) gegebenenfalls Zerkleinerung des Polymergels;
iii) Trocknung des gegebenenfalls zerkleinerten Polymergels unter Erhalt wasserabsorbierender Polymergebilde, sowie
iv) gegebenenfalls Oberflächennachbehandlung der wasserabsorbierenden Polymergebilde.

5. Verfahren nach einem der Ansprüche 2 bis 4, wobei die Acrylsäure zu mindestens 20 mol-%, bezogen auf das Monomer, als Salz vorliegt.

6. Verfahren nach einem der vorhergehenden Ansprüche, wobei das Glycerin aus der Verseifung von Fetten gewonnen wird.

7. Verfahren nach einem der vorhergehenden Ansprüche, wobei Glycerin bei der Erzeugung von flüssigen Brennstoffen aus natürlichen Rohstoffen anfällt.

8. Verfahren nach einem der vorhergehenden Ansprüche, wobei die Dehydratisierung mindestens teilweise in flüssiger Phase erfolgt.

9. Verfahren nach einem der Ansprüche 1 bis 11, wobei die Dehydratisierung mindestens teilweise in einer Gasphase erfolgt.

10. Verfahren nach einem der vorhergehenden Ansprüche, wobei das Glycerin in wässriger Phase eingesetzt wird.

11. Verfahren nach einem der vorhergehenden Ansprüche, wobei ein Dehydratisierungskatalysator eingesetzt wird.

12. Verfahren nach Anspruch 11, wobei der Dehydratisierungskatalysator mit einem Träger x. in Kontakt steht.

13. Verfahren nach Anspruch 12, wobei der Träger x. ein Feststoff mit einem Gesamtporenvolumen nach DIN 66133 in einem Bereich von 0,1 bis 1,5 ml/g ist.

14. Verfahren nach einem der Ansprüche 11 bis 13, wobei der Dehydratisierungskatalysator eine anorganische Säure ist.

15. Verfahren nach Anspruch 14, wobei der Dehydratisierungskatalysator in einer Lösung vorliegt.

16. Verfahren nach einem der vorhergehenden Ansprüche, wobei die Gasphasenoxidation in Gegenwart eines ein Übergangsmetall in elementare oder in chemisch gebundener Form oder beides aufweisenden Oxidationskatalysators erfolgt.

17. Verfahren nach einem der vorhergehenden Ansprüche, wobei das Dehydratisierungsprodukt in einer wässrigen Phase der Gasphasenoxidation zugeführt wird.

18. Verfahren nach einem der vorhergehenden Ansprüche, wobei die Gasphasenoxidation in einem Temperaturbereich von 200 bis 400°C erfolgt.

19. Verfahren nach einem der vorhergehenden Ansprüche, wobei das Monomergas die Acrylsäure in einer Menge im Bereich von 5 bis 50 Gew.-%, jeweils bezogen auf das Monomergas, beinhaltet.

20. Verfahren nach einem der vorhergehenden Ansprüche, wobei das Quenchmittel Wasser oder eine organische Verbindung mit einen Siedepunkt im Bereich von 50 bis 250°C oder Wasser und diese organische Verbindung beinhaltet.

21. Verfahren nach einem der vorhergehenden Ansprüche, wobei die Quenchphase die Acrylsäure in einer Menge im Bereich von 30 bis 90 Gew.-%, jeweils bezogen auf Monomerphase, beinhaltet.

22. Verfahren nach einem der vorhergehenden Ansprüche, wobei das Aufarbeiten bei Temperaturen unterhalb des Siedepunkts der Acrylsäure erfolgt.

23. Verfahren nach einem der vorhergehenden Ansprüche, wobei das Aufarbeiten durch Extraktion oder Kristallisation oder beides erfolgt.

24. Vorrichtung zur Herstellung von Acrylsäure fluidleitend miteinander verbunden aufweisend
1a. einen Dehydratisierungsreaktor (2),
2a. einen Gasphasenoxidationsreaktor (3),
3a. eine Quencheinheit (4),
4a. eine Aufarbeitungseinheit (5).

25. Vorrichtung zur Herstellung von Polymeren fluidleitend miteinander verbunden aufweisend
1b. einen Dehydratisierungsreaktor (2),
2b. einen Gasphasenoxidationsreaktor (3),
3b. eine Quencheinheit (4),
4b. eine Aufarbeitungseinheit (5),
5b. eine Polymerisationseinheit (6).

26. Vorrichtung nach Anspruch 24 oder 25, wobei der Dehydratisierungsreaktor (2)
- einen Eduktbehälter (7), gefolgt von
- einem ein Katalysator (8) aufnehmenden den Reaktionsbereich (9), gefolgt von
- einem Quencher (10) mit einer Ableitung (11) zu dem Gasphasenoxidationsreaktor (3)
aufweist.

27. Vorrichtung nach Anspruch 26, wobei nach dem Eduktbehälter (7) und vor dem Reaktionsbereich (9) ein Verdampfer (12) vorgesehen ist.

28. Vorrichtung nach Anspruch 27, wobei der Reaktionsbereich (9) einen unteren Produktauslass (13) aufweist, der in den Quencher (10) mündet.

29. Vorrichtung nach Anspruch 28, wobei der Reaktionsbereich (9) einen oberen Produktauslass (14) aufweist, der in den Quencher (10) mündet.

30. Verfahren nach einem der Ansprüche 1 und 5 bis 23, wobei das Verfahren in einer Vorrichtung nach einem der Ansprüche 24 und 26 bis 29 durchgeführt wird.

31. Verfahren nach einem der Ansprüche 2 bis 23, wobei das Verfahren in einer Vorrichtung nach einem der Ansprüche 24 bis 29 durchgeführt wird.

## Claims

1. Process for producing acrylic acid, comprising at least the steps of:
a. dehydrating glycerol to a dehydration product comprising acrolein;
b. gas phase oxidizing the dehydration product to obtain a monomer gas comprising acrylic acid;
c. contacting the monomer gas with a quench agent to obtain a quench phase comprising acrylic acid;
d. working up the quench phase to obtain a monomer phase comprising acrylic acid.

2. Process for producing a polymer by free radical polymerization of acrylic acid, comprising at least the steps of:
A. dehydrating glycerol to a dehydration product comprising acrolein;
B. gas phase oxidizing the dehydration product to obtain a monomer gas comprising acrylic acid;
C. contacting the monomer gas with a quench agent to obtain a quench phase comprising acrylic acid;
D. working up the quench phase to obtain a monomer phase comprising acrylic acid;
E. free-radically polymerizing the monomer phase.

3. Process according to Claim 2 wherein the polymer is a water-absorbing polymeric structure.

4. Process according to Claim 3 wherein the water-absorbing polymeric structure is obtainable by a process comprising the steps of:
i) polymerizing the acrylic acid in the presence of a crosslinker to obtain a polymer gel;
ii) if appropriate comminuting the polymer gel;
iii) drying the comminuted or uncomminuted polymer gel to obtain water-absorbing polymeric structures, and
iv) if appropriate surface aftertreatment of the water-absorbing polymeric structures.

5. Process according to any one of the Claims 2 to 4 wherein at least 20 mol% of the acrylic acid, based on the monomer, is present as salt.

6. Process according to any one of the preceding claims wherein the glycerol is obtained from the saponification of fats.

7. Process according to any one of the preceding claims wherein glycerol is generated in the production of liquid fuels from natural raw materials.

8. Process according to any one of the preceding claims wherein the dehydrating is at least partly effected in liquid phase.

9. Process according to any one of the Claims 1 to 11 wherein the dehydrating is at least partly effected in a gas phase.

10. Process according to any one of the preceding claims wherein the glycerol is used in aqueous phase.

11. Process according to any one of the preceding claims wherein a dehydration catalyst is used.

12. Process according to Claim 11 wherein the dehydration catalyst is in contact with a support x.

13. Process according to Claim 12 wherein the support x. is a solid having a DIN 66133 total pore volume ranging from 0.1 to 1.5 ml/g.

14. Process according to any one of the Claims 11 to 13 wherein the dehydration catalyst is an inorganic acid.

15. Process according to Claim 14 wherein the dehydration catalyst is present in a solution.

16. Process according to any one of the preceding claims wherein the gas phase oxidation is effected in the presence of an oxidation catalyst comprising a transition metal in elemental form or in chemically bound form or both.

17. Process according to any one of the preceding claims wherein the dehydration product is fed to the gas phase oxidation in an aqueous phase.

18. Process according to any one of the preceding claims wherein the gas phase oxidation is effected in a temperature range from 200 to 400°C.

19. Process according to any one of the preceding claims wherein the monomer gas includes the acrylic acid in an amount ranging from 5% to 50% by weight, both based on the monomer gas.

20. Process according to any one of the preceding claims wherein the quench agent comprises water or an organic compound having a boiling point in the range from 50 to 250°C or water and this organic compound.

21. Process according to any one of the preceding claims wherein the quench phase includes the acrylic acid in an amount ranging from 30% to 90% by weight, both based on monomer phase.

22. Process according to any one of the preceding claims wherein the working up is effected at temperatures below the boiling point of acrylic acid.

23. Process according to any one of the preceding claims wherein the working up is effected by extraction or crystallization or both.

24. Apparatus for producing acrylic acid, comprising in fluid-conducting communication with one another:
1a. a dehydration reactor (2),
2a. a gas phase oxidation reactor (3),
3a. a quenching unit (4),
4a. a workup unit (5).

25. Apparatus for producing polymers, comprising in fluid-conducting communication with one another:
1b. a dehydration reactor (2),
2b. a gas phase oxidation reactor (3),
3b. a quenching unit (4),
4b. a workup unit (5),
5b. a polymerization unit (6).

26. Apparatus according to Claim 24 or 25 wherein the dehydratiant (2) comprises
- a reactant container (7), followed by
- a reaction region (9) accommodating a catalyst (8), followed by
- a quencher (10) having an exit line (11) to the gas phase oxidation reactor (3).

27. Apparatus according to Claim 26 wherein a vaporizer (12) is provided downstream of the reactant container (7) and upstream of the reaction region (9).

28. Apparatus according to Claim 27 wherein the reaction region (9) comprises a lower product outlet (13) which empties into the quencher (10).

29. Apparatus according to Claim 28 wherein the reaction region (9) comprises an upper product outlet (14) which empties into the quencher (10).

30. Process according to any one of the Claims 1 and 5 to 23, conducted in apparatus according to any one of the Claims 24 and 26 to 29.

31. Process according to any one of the Claims 2 to 23, conducted in apparatus according to any one of the Claims 24 to 29.

## Revendications

1. Procédé pour la préparation d'acide acrylique, présentant au moins les étapes suivantes de :
a. déshydratation de glycérol en un produit de déshydratation présentant de l'acroléine ;
b. oxydation en phase gazeuse du produit de déshydratation avec obtention d'un gaz monomère présentant de l'acide acrylique ;
c. mise en contact du gaz monomère avec un agent de refroidissement brusque avec obtention d'une phase de refroidissement brusque présentant de l'acide acrylique ;
d. traitement de la phase de refroidissement brusque avec obtention d'une phase monomère contenant de l'acide acrylique.

2. Procédé pour la préparation d'un polymère par polymérisation par voie radicalaire d'acide acrylique, présentant au moins les étapes suivantes de :
A. déshydratation de glycérol en un produit de déshydratation présentant de l'acroléine ;
B. oxydation en phase gazeuse du produit de déshydratation avec obtention d'un gaz monomère présentant de l'acide acrylique ;
C. mise en contact du gaz monomère avec un agent de refroidissement brusque avec obtention d'une phase de refroidissement brusque présentant de l'acide acrylique ;
D. traitement de la phase de refroidissement brusque avec obtention d'une phase monomère contenant de l'acide acrylique ;
E. polymérisation par voie radicalaire de la phase monomère.

3. Procédé selon la revendication 2, le polymère étant une structure polymère absorbant l'eau.

4. Procédé selon la revendication 3, la structure polymère absorbant l'eau pouvant être obtenue par un procédé comprenant les étapes de procédé suivantes de :
i) polymérisation de l'acide acrylique en présence d'un réticulant avec formation d'un gel polymère ;
ii) le cas échéant fragmentation du gel polymère ;
iii) séchage du gel polymère le cas échéant fragmenté avec obtention de structures polymères absorbant l'eau, ainsi que
iv) le cas échéant posttraitement de surface des structures polymères absorbant l'eau.

5. Procédé selon l'une quelconque des revendications 2 à 4, l'acide acrylique se trouvant à raison d'au moins 20% en mole, par rapport au monomère, sous forme de sel.

6. Procédé selon l'une quelconque des revendications précédentes, le glycérol étant obtenu à partir de la saponification de graisses.

7. Procédé selon l'une quelconque des revendications précédentes, le glycérol étant obtenu lors de la production de combustibles liquides à partir de matières premières naturelles.

8. Procédé selon l'une quelconque des revendications précédentes, la déshydratation ayant lieu au moins partiellement en phase liquide.

9. Procédé selon l'une quelconque des revendications 1 à 11, la déshydratation ayant lieu au moins partiellement dans une phase gazeuse.

10. Procédé selon l'une quelconque des revendications précédentes, le glycérol étant utilisé en phase aqueuse.

11. Procédé selon l'une quelconque des revendications précédentes, un catalyseur de déshydratation étant utilisé.

12. Procédé selon la revendication 11, le catalyseur de déshydratation étant en contact avec un support x.

13. Procédé selon la revendication 12, le support x étant un solide présentant un volume total de pores selon la norme DIN 66133 dans une plage de 0,1 à 1,5 ml/g.

14. Procédé selon l'une quelconque des revendications 11 à 13, le catalyseur de déshydratation étant un acide inorganique.

15. Procédé selon la revendication 14, le catalyseur de déshydratation se trouvant dans une solution.

16. Procédé selon l'une quelconque des revendications précédentes, l'oxydation en phase gazeuse ayant lieu en présence d'un catalyseur d'oxydation présentant un métal de transition sous forme élémentaire ou sous forme chimiquement liée ou les deux.

17. Procédé selon l'une quelconque des revendications précédentes, le produit de déshydratation étant introduit dans une phase aqueuse dans l'oxydation en phase gazeuse.

18. Procédé selon l'une quelconque des revendications précédentes, l'oxydation en phase gazeuse étant réalisée dans une plage de température de 200 à 400°C.

19. Procédé selon l'une quelconque des revendications précédentes, le gaz monomère contenant l'acide acrylique en une quantité dans la plage de 5 à 50% en poids, à chaque fois par rapport au gaz monomère.

20. Procédé selon l'une quelconque des revendications précédentes, l'agent de refroidissement brusque comprenant de l'eau ou un composé organique présentant un point d'ébullition dans la plage de 50 à 250°C ou de l'eau et ce composé organique.

21. Procédé selon l'une quelconque des revendications précédentes, la phase de refroidissement brusque contenant l'acide acrylique en une quantité dans la plage de 30 à 90% en poids, à chaque fois par rapport à la phase monomère.

22. Procédé selon l'une quelconque des revendications précédentes, le traitement ayant lieu à des températures inférieures au point d'ébullition de l'acide acrylique.

23. Procédé selon l'une quelconque des revendications précédentes, le traitement ayant lieu par extraction ou par cristallisation ou les deux.

24. Dispositif pour la préparation d'acide acrylique présentant, en communication fluidique les uns avec les autres,
1a. un réacteur de déshydratation (2),
2a. un réacteur d'oxydation en phase gazeuse (3),
3a. une unité de refroidissement brusque (4),
4a. une unité de traitement (5).

25. Dispositif pour la préparation de polymères présentant, en communication fluidique les uns avec les autres,
1b. un réacteur de déshydratation (2),
2b. un réacteur d'oxydation en phase gazeuse (3),
3b. une unité de refroidissement brusque (4),
4b. une unité de traitement (5),
5b. une unité de polymérisation (6).

26. Dispositif selon la revendication 24 ou 25, le réacteur de déshydratation (2) présentant
- un récipient (7) à produit de départ, suivi
- d'une zone de réaction (9) reprenant un catalyseur (8), suivie
- d'un dispositif (10) de refroidissement brusque présentant une conduite (11) vers le réacteur (3) d'oxydation en phase gazeuse.

27. Dispositif selon la revendication 26, un évaporateur (12) étant disposé en aval du récipient (7) à produit de départ et en amont de la zone de réaction (9).

28. Dispositif selon la revendication 27, la zone de réaction (9) présentant une sortie (13) inférieure de produit qui débouche dans le dispositif (10) de refroidissement brusque.

29. Dispositif selon la revendication 28, la zone de réaction (9) présentant une sortie (14) supérieure de produit qui débouche dans le dispositif (10) de refroidissement brusque.

30. Procédé selon l'une quelconque des revendications 1 et 5 à 23, le procédé étant réalisé dans un dispositif selon l'une quelconque des revendications 24 et 26 à 29.

31. Procédé selon l'une quelconque des revendications 2 à 23, le procédé étant réalisé dans un dispositif selon l'une quelconque des revendications 24 à 29.
